# EUROPEAN PATENT APPLICATION

(11) **EP 3 674 287 A1**
(43) Date of publication of application: **01.07.2020**
(21) Application number: 18460088.0
(22) Date of filing: 31.12.2018
(51) Int. Cl.: C07C 229/34, C07C 227/42, C07C 57/44, C07C 63/06, C07C 65/10, A61P 21/00, A61K 31/197

(54) **COCRYSTALS OF (R)-BACLOFEN**

(71) Applicant: Zaklady Farmaceutyczne "Polpharma" S.A., 83-200 Starogard Gdanski (PL)
(72) Inventor: Tesson, Nicolas, 08028 Barcelona (ES); Jiménez González, Carmen, 08028 Barcelona (ES); Nienaltowski, Tomasz, 18-400 Lomza (PL); Pogoda, Dorota, 51-649 Wroclaw (PL); Krzyzanowski, Mariusz, 83-220 Skórcz (PL); Pawlowska, Jolanta, 83-200 Starogard Gdanski (PL)
(74) Representative: Rechnio, Justyna Tatiana

(57) **Abstract**

The invention relates to novel cocrystals of (R)-Baclofen and processes for preparation thereof, and in particular to cocrystals of (R)-Baclofen with cinnamic acid, benzoic acid, salicylic acid and ferulic acid. It also refers to pharmaceutical compositions containing said cocrystals and to a use of said cocrystals or pharmaceutical compositions for the treatment of a disease or disorder selected from spasticity due to multiple sclerosis, spinal cord injury or cerebral palsy, and alcoholism.

## Description

The invention relates to novel cocrystals of (R)-Baclofen and processes for preparation thereof. It also refers to pharmaceutical compositions containing said cocrystals and to a use of said cocrystals or pharmaceutical compositions for the treatment of a disease or disorder selected from spasticity due to multiple sclerosis, spinal cord injury or cerebral palsy, and alcoholism.

### Background art

(R)-Baclofen, i.e. (3R)-4-amino-3-(4-chlorophenyl)butanoic acid, has the following structural formula (1).

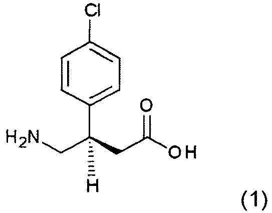

(R)-Baclofen is an antispastic drug and an analog of the neurotransmitter 4-aminobutyric acid (GABA).

4-Aminobutyric acid (GABA) is the major inhibitory neurotransmitter in the mammalian central nervous system (CNS), and it operates through ionotropic (GABA_{A} and GABA_{C}) as well as G protein-coupled (GABA_{B}) receptors. The dysfunction of the central GABA system is responsible for the development and outbreak of epilepsy, Huntington's and Parkinson's diseases, and other psychiatric disorders, such as anxiety and pain. Baclofen interacts stereospecifically with GABA_{B} receptor and is a selective and therapeutically available GABA_{B} agonist. It is used in the treatment of paroxysmal pain of trigeminal neuralgia as well as spasticity of spinal cord, a serious disease characterized by an increase in muscle tone usually perceived as muscle tightness or achiness in limbs. Although commercialized in its racemic form, the (R)-(-) isomer has been found to be the more active one and patients should greatly benefit from the administration of enantiomerically pure (R)-Baclofen.

US2009/0137819 discloses the process of preparation of optically active 4-amino-3-(4-chlorophenyl)butanoic acid by adding an aqueous solution of sodium hydroxide into an aqueous solution of a hydrochloride salt of optically active 4-amino-3-(4-chlorophenyl)butanoic acid to attain neutralizing crystallization. M. R. Caira et al. (in Optical resolution of Baclofen via diastereomeric salt pair formation between 3-(p-chlorophenyl)glutaramic acid and (S)-(-)-α-phenylethylamine, J. Chem. Soc., Perkin Transactions 2, 1997, pp. 763-768) disclose a method in which the same salt is yielded by adding diluted hydrochloric acid to an aqueous hydroxide solution of optically active 4-amino-3-(4-chlorophenyl)butanoic acid to attain neutralizing crystallization. Caira *et al.* also disclose that the crystals are further heated in methanol.

EP2345634 B1 discloses crystal forms of (R)-Baclofen, i.e. crystal form A and form B. Crystal form B may be produced by the known processes described by US2009/0137819 or Caira et al.

Crystal form A is obtained from (R)-Baclofen crystal form B by stirring crystal form B in water at the pH in the range of 7-8 for 1 hour at 65°C and then cooling down the solution. The cooled mixture is filtered off to obtain the crystal form A.

EP2345634 B1 further discloses that in the DSC measurement the crystal form A has a higher endothermic peak and a higher exothermic peak than crystal form B, crystal form A is far better in thermal stability than crystal form B. Moreover, it is also considered that the crystal form A is better in storage stability than crystal form B.

The solubility of crystal form A in 100 g of water ranges from 0.7 to 1.1 g (0.9±0.2 g) at 25°C. On the other hand, the solubility of crystal form B in 100 g of water ranges from 1.8 to 2.2 g (2.0 ± 0.2 g) at 25°C, which according to USP solubility test (General Notices to point 5.30. Description and Solubility), is sparingly soluble in water.

There is a continuous demand in the pharmaceutical industry to be able to produce pure, morphologically homogenous and stable forms of active substances. It is also well known that solubility, dissolution and gastrointestinal permeability are fundamental parameters that control rate and extent of drug absorption and its bioavailability. The aqueous solubility of a drug is a fundamental property that plays an important role in the absorption of the drug after oral administration. It also governs the possibility of parenteral administration of a drug and is useful in manipulating and testing of drug properties during the drug design and development process. The drug solubility is an equilibrium measure, but also the dissolution rate at which the solid drug or drug from the dosage form passes into solution is critically important when the dissolution time is limited. Poor aqueous solubility and poor dissolution in the gastrointestinal fluids are limiting factors to the *in vivo* bioavailability after oral administration. Therefore, *in vitro* dissolution has been recognized as an important element in drug development and thus increasing the dissolution rate of poorly soluble drugs and enhancing their bioavailability is an important challenge to pharmaceutical scientists. Furthermore, from the point of view of the cost-effectiveness of production it is extremely important to produce the product using a procedure that may also be realized on the industrial scale that is easy to reproduce and results in a morphologically homogeneous, contaminant-free, and a stable form.

It is well-known that the successful formation of cocrystals is neither predictable nor otherwise guaranteed. Furthermore, computational analysis cannot predict the structure or properties of cocrystals. Of particular importance is the fact that cocrystal formation is a new field where there is generally not yet much experience and knowledge available in the art. As such, general trends are not yet evident. It is also expected that the change in properties when comparing a drug polymorph with cocrystals will be far more drastic as compared with the variance in properties amongst polymorphs. This means that the chances of success for providing cocrystals having above mentioned properties, in the present case were generally low.

It can also be expected that processes for producing cocrystals, in particular in large scale applications, will require different approaches as those which have been developed in the field of polymorphs. At present the number of drug cocrystals in pharmaceutics on the market, if any, is low.

Accordingly, the objective underlying the present invention was to improve the aqueous solubility of (R)-Baclofen as well as the chemical and the crystalline stability with respect to the known crystal forms A and B of (R)-Baclofen.

Our goal was to produce a new highly pure and stable, contaminant-free form of (R)-Baclofen with homogeneous morphology, which provides high aqueous solubility and thermal stability and is producible at industrial scale.

It has unexpectedly been found that it is possible to provide cocrystals of (R)-Baclofen which are morphologically homogeneous, contaminant-free, stable and in particular have high aqueous solubility and thermal stability.

### Summary of the invention

The invention relates to a cocrystal of (R)-Baclofen, i.e. (3R)-4-amino-3-(4-chlorophenyl)-butanoic acid, with a coformer, and in particular to cocrystal of (R)-Baclofen with the coformer selected from the group consisting of cinnamic acid ((2E)-3-phenylprop-2-enoic acid), benzoic acid (benzene carboxylic acid), salicylic acid (2-hydroxybenzoic acid) and ferulic acid ((E)-3-(4-hydroxy-3-methoxy-phenyl)prop-2-enoic acid), and to processes for manufacturing thereof. It also refers to pharmaceutical compositions comprising said cocrystals. The invention also relates to said pharmaceutical compositions for the treatment of a disease or disorder defined in the claims.

### List of figures

- Figure 1:: shows a representative XRPD pattern of (R)-Baclofen-cinnamic acid cocrystal Form III.
- Figure 2:: shows a representative DSC plot of (R)-Baclofen-cinnamic acid cocrystal Form III.
- Figure 3:: shows a representative XRPD pattern of (R)-Baclofen-cinnamic acid cocrystal Form IV.
- Figure 4:: shows a representative DSC plot of (R)-Baclofen-cinnamic acid cocrystal Form IV.
- Figure 5:: shows a representative XRPD pattern of (R)-Baclofen-benzoic acid cocrystal Form II.
- Figure 6:: shows a representative DSC plot of (R)-Baclofen-benzoic acid cocrystal Form II.
- Figure 7:: shows a representative XRPD pattern of (R)-Baclofen-salicylic acid cocrystal Form VIII.
- Figure 8:: shows a representative DSC plot of (R)-Baclofen-salicylic acid cocrystal Form VIII.
- Figure 9:: shows a representative XRPD pattern of (R)-Baclofen-ferulic acid cocrystal Form XII.
- Figure 10:: shows a representative DSC plot of (R)-Baclofen-ferulic acid cocrystal Form XII.
- Figure 11:: shows ¹H NMR spectrum of (R)-Baclofen-cinnamic acid cocrystal Form III.
- Figure 12:: shows ¹H NMR spectrum of (R)-Baclofen-cinnamic acid cocrystal Form IV.
- Figure 13:: shows ¹H NMR spectrum of (R)-Baclofen-benzoic acid cocrystal Form II.
- Figure 14:: shows ¹H NMR spectrum of (R)-Baclofen-salicylic acid cocrystal Form VIII.
- Figure 15:: shows ¹H NMR spectrum of (R)-Baclofen-ferulic acid cocrystal Form XII.

### Detailed description of the invention

The present invention relates to a cocrystal of (R)-Baclofen, having structural formula (1) 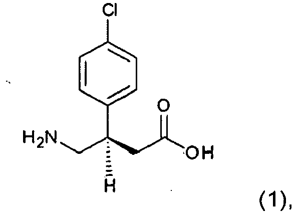 with a coformer. The coformer according to the invention is selected from the group consisting of cinnamic acid, benzoic acid, salicylic acid and ferulic acid.

It has unexpectedly been found that it is possible to provide cocrystals of (R)-Baclofen with cinnamic acid, benzoic acid, salicylic acid and ferulic acid which are morphologically homogeneous, contaminant-free, stable and in particular have high aqueous solubility and thermal stability.

A preliminary study of (R)-Baclofen solubility with different solvents (see Example 26 below) allowed to design a proper experimental space to obtain or detect new crystalline form and to reach the following conclusions as to solubility of (R)-Baclofen:
- (R)-Baclofen has low solubility in water and majority of commonly used organic solvents;
- (R)-Baclofen is soluble at reflux temperature in DMSO, DMF and water;
- (R)-Baclofen is insoluble in the other tested solvents even at reflux temperature.

For the cocrystal screening the present inventors selected 2 techniques (slurrying and grinding), 18 solvents and (R)-Baclofen /coformer molar ratios of 1:1, 1:2, 1:3, 1:5 and 2:3.

The selected coformers used for cocrystal screening are summarized in Table 1.

**Table 1: List of screened coformers**

| **Coformer** | **CAS Number** |
|---|---|
| Benzoic acid | 65-85-0 |
| Cinnamic acid | 140-10-3 |
| Gentisic acid | 490-79-9 |
| 4-Aminobenzoic acid | 150-13-0 |
| Vanillic acid | 121-34-6 |
| p-Anisic acid | 100-09-4 |
| Citric acid | 77-92-9 |
| Gallic acid | 149-91-7 |
| L-Ascorbic acid | 50-81-7 |
| Fumaric acid | 110-17-8 |
| Salicylic acid | 69-72-7 |
| Vanillin | 121-33-5 |
| Maltol | 118-71-8 |
| Ferulic acid | 1135-24-6 |

Combining the above parameters, 237 representative experiments were performed. From this screening, new cocrystal forms were detected by XRPD and the results are summarized in Table 2.

**Table 2: New cocrystals of (R)-Baclofen**

| **Coformer** | **Cocrystal Form** |
|---|---|
| Benzoic acid | Form II |
| Cinnamic acid | Forms III and IV |
| Fumaric acid | Form VII |
| Salicylic acid | Form VIII |
| Vanillin | Form IX |
| Ferulic acid | Form XII |

The cocrystals disclosed herein provide various benefits over the known forms of (R)-Baclofen, in particular its known crystal forms A and B.

First aspect of the present invention is directed to the cocrystal of (R)-Baclofen with cinnamic acid.

The molar ratio of (R)-Baclofen to cinnamic acid in the above cocrystal is about 1:1.

In the preferred embodiment of the above aspect, the present invention relates to a cocrystal of (R)-Baclofen with cinnamic acid Form III, further: (R)-Baclofen-cinnamic acid cocrystal Form III or cocrystal Form III, which is a morphologically homogeneous, contaminant-free and stable form. In particular, it has high aqueous solubility and thermal stability when compared to prior art crystal forms A and B of (R)-Baclofen.

The (R)-Baclofen-cinnamic acid cocrystal Form III of the present invention has an X-ray powder diffraction pattern with peaks at 2-theta angles of 4.60±0.2 degrees, 9.03±0.2 degrees, 13.51±0.2 degrees, 17.99±0.2 degrees, 19.11±0.2 degrees, 19.77±0.2 degrees, 20.65±0.2 degrees, and 23.90±0.2 degrees, when using Cu-Ka radiation. Details of the X-ray powder diffraction measurement are provided further, in the experimental part.

In more detail, the cocrystal Form III of the present disclosure, has an X-ray powder diffraction pattern with peaks and relative intensities as shown in Table 3.

**Table 3: XRPD data of (R)-Baclofen-cinnamic acid cocrystal Form III**

| **No.** | **2-theta (deg)** | **d-spacing (Å)** | **Relative intensity (%)** |
|---|---|---|---|
| 1 | 4.6034(8) | 19.180(3) | 100.00 |
| 2 | 5.41(4) | 16.31(13) | 1.85 |
| 3 | 9.0380(16) | 9.7766(17) | 17.52 |
| 4 | 11.969(8) | 7.388(5) | 0.26 |
| 5 | 12.854(19) | 6.882(10) | 0.65 |
| 6 | 13.5154(14) | 6.5462(7) | 41.46 |
| 7 | 13.788(12) | 6.418(6) | 0.54 |
| 8 | 14.335(13) | 6.174(6) | 1.27 |
| 9 | 14.63(4) | 6.052(16) | 0.49 |
| 10 | 15.167(17) | 5.837(7) | 1.42 |
| 11 | 15.519(6) | 5.705(2) | 3.52 |
| 12 | 17.646(11) | 5.022(3) | 3.45 |
| 13 | 17.999(6) | 4.9242(16) | 6.51 |
| 14 | 18.706(7) | 4.7399(17) | 49.92 |
| 15 | 19.1137(16) | 4.6396(4) | 52.96 |
| 16 | 19.7713(17) | 4.4867(4) | 60.34 |
| 17 | 20.211(9) | 4.3902(19) | 9.49 |
| 18 | 20.651(3) | 4.2975(6) | 30.02 |
| 19 | 21.728(8) | 4.0869(14) | 7.06 |
| 20 | 22.047(7) | 4.0285(13) | 0.92 |
| 21 | 22.546(8) | 3.9404(14) | 6.13 |
| 22 | 22.991(8) | 3.8652(14) | 5.65 |
| 23 | 23.903(8) | 3.7198(12) | 11.82 |
| 24 | 24.415(13) | 3.6429(19) | 0.76 |
| 25 | 25.415(7) | 3.5017(9) | 33.28 |
| 26 | 25.772(16) | 3.454(2) | 22.93 |
| 27 | 25.945(17) | 3.431(2) | 5.21 |
| 28 | 26.35(2) | 3.379(3) | 4.72 |
| 29 | 26.922(9) | 3.3091(11) | 13.95 |
| 30 | 27.648(10) | 3.2238(11) | 17.46 |
| 31 | 28.93(2) | 3.084(2) | 6.08 |
| 32 | 29.27(3) | 3.049(3) | 2.99 |
| 33 | 30.246(11) | 2.9525(10) | 13.39 |
| 34 | 31.01(7) | 2.881(6) | 5.54 |
| 35 | 31.686(11) | 2.8216(10) | 17.06 |
| 36 | 32.23(4) | 2.775(3) | 2.12 |
| 37 | 32.43(9) | 2.759(8) | 5.18 |
| 38 | 32.951(18) | 2.7161(15) | 1.79 |
| 39 | 34.14(2) | 2.6245(17) | 6.57 |
| 40 | 34.59(5) | 2.591(4) | 3.31 |
| 41 | 34.92(4) | 2.568(3) | 3.28 |
| 42 | 35.40(9) | 2.533(6) | 1.09 |

Furthermore, the cocrystal Form III of the present invention has a single endothermic peak in a differential scanning calorimetry plot with onset at 163.5± 1 °C.

Details of the differential scanning calorimetry measurement are provided further, in the experimental part.

Preferably, the cocrystal Form III of the present invention is substantially chemically pure, i.e. it contains less than 1 area-%, or less than 0.5 area-%, preferably less than 0.1 area-%, of compounds other than (R)-Baclofen and cinnamic acid as determined by high performance liquid chromatography (HPLC).

Furthermore, the cocrystal Form III of the present invention is substantially polymorphically pure, and preferably contains less than 5 wt.-% of other crystalline, non-crystalline or amorphous forms of (R)-Baclofen, cinnamic acid and cocrystals thereof.

The present invention relates also to a process for preparing (R)-Baclofen-cinnamic acid cocrystal Form III as characterized above, wherein said process comprises the following steps:
a) contacting (R)-Baclofen and cinnamic acid in a molar ratio of (R)-Baclofen to cinnamic acid within a range 1:1-1:5 with a solvent selected from water, acetonitrile, methyl isobutyl ketone, heptane, cyclohexane, dimethylformamide, ethyl acetate, methanol, ethanol, isopropanol, dioxane, preferably with ethyl acetate,
b) optionally, seeding a mixture of step a) with pre-existing crystals of Form III,
c) crystallising the (*R*)-Baclofen-cinnamic acid cocrystal Form III.

In the second embodiment the first aspect, the present invention relates to a cocrystal of (R)-Baclofen with cinnamic acid Form IV, further: (R)-Baclofen-cinnamic acid cocrystal Form IV or cocrystal Form IV, which is a morphologically homogeneous, contaminant-free and stable form. In particular, it has high aqueous solubility and thermal stability when compared to prior art crystal forms A and B of (R)-Baclofen.

The cocrystal Form IV of the present invention has an X-ray powder diffraction pattern with peaks at 2-theta angles of 5.15±0.2 degrees, 10.27±0.2 degrees, 12.84±0.2 degrees, 13.10±0.2 degrees, 15.42±0.2 degrees, 18.58±0.2 degrees, 21.14±0.2 degrees, 24.53±0.2 degrees, 25.34±0.2 degrees, and 27.14±0.2 degrees, when using Cu-Ka radiation. Further details of X-ray powder diffraction measurement are provided herein below.

In more detail, the cocrystal Form IV of the present disclosure, has an X-ray powder diffraction pattern with peaks and relative intensities as shown in Table 4.

**Table 4: XRPD of (R)-Baclofen-cinnamic acid cocrystal Form IV**

| **No.** | **2-theta (deg)** | **d-spacing (Å)** | **Relative intensity (%)** |
|---|---|---|---|
| 1 | 5.1508(19) | 17.143(6) | 100.00 |
| 2 | 10.279(5) | 8.599(4) | 2.48 |
| 3 | 12.843(5) | 6.887(3) | 5.48 |
| 4 | 13.102(6) | 6.752(3) | 6.23 |
| 5 | 14.33(12) | 6.18(5) | 0.53 |
| 6 | 15.428(2) | 5.7388(8) | 36.97 |
| 7 | 17.28(2) | 5.128(6) | 12.42 |
| 8 | 17.673(6) | 5.0144(16) | 10.75 |
| 9 | 18.582(2) | 4.7711(6) | 37.61 |
| 10 | 19.09(3) | 4.646(6) | 8.77 |
| 11 | 19.25(2) | 4.606(5) | 2.61 |
| 12 | 21.142(3) | 4.1989(5) | 41.21 |
| 13 | 21.6200(16) | 4.1071(3) | 62.73 |
| 14 | 21.901(6) | 4.0550(10) | 2.38 |
| 15 | 22.630(7) | 3.9261(12) | 5.53 |
| 16 | 23.660(9) | 3.7573(14) | 6.66 |
| 17 | 24.531(3) | 3.6258(4) | 30.95 |
| 18 | 25.345(4) | 3.5113(6) | 25.74 |
| 19 | 25.830(4) | 3.4464(6) | 29.61 |
| 20 | 25.996(4) | 3.4248(5) | 46.33 |
| 21 | 27.140(3) | 3.2829(4) | 27.69 |
| 22 | 27.363(16) | 3.2568(18) | 6.38 |
| 23 | 27.872(8) | 3.1984(9) | 6.64 |
| 24 | 28.346(8) | 3.1460(9) | 4.89 |
| 25 | 28.87(2) | 3.090(2) | 0.51 |
| 26 | 29.54(2) | 3.021(2) | 2.63 |
| 27 | 30.48(2) | 2.9303(18) | 1.94 |
| 28 | 31.10(2) | 2.873(2) | 1.02 |
| 29 | 32.283(14) | 2.7707(11) | 1.24 |
| 30 | 32.880(8) | 2.7218(6) | 5.14 |
| 31 | 33.287(7) | 2.6894(6) | 4.94 |
| 32 | 33.96(4) | 2.638(3) | 0.52 |
| 33 | 34.350(9) | 2.6086(6) | 0.47 |
| 34 | 35.659(7) | 2.5158(5) | 6.18 |

Furthermore, the cocrystal Form IV of the present invention has a single endothermic peak in a differential scanning calorimetry plot with an onset at 161.2 ± 1°C. Details of the differential scanning calorimetry measurement are provided further, in the experimental part.

Furthermore, the cocrystal Form IV of the present invention is substantially chemically pure, i.e. contains less than 1 area-%, or less than 0.5 area-%, preferably less than 0.1 area-%, of compounds other than (R)-Baclofen and cinnamic acid as determined by high performance liquid chromatography (HPLC).

Furthermore, the cocrystal Form IV of the present invention is substantially polymorphically pure, and preferably contains less than 5 wt.-% of other crystalline, non-crystalline or amorphous forms of (R)-Baclofen acid, cinnamic acid and cocrystals thereof.

The present invention relates also to a process for preparing (R)-Baclofen-cinnamic acid cocrystal Form IV as characterized above, wherein said process comprises the following steps:
a) contacting (R)-Baclofen and cinnamic acid in a molar ratio of (R)-Baclofen to cinnamic acid within a range 1:1-1:5 with acetone,
b) optionally, seeding a mixture of step a) with pre-existing crystals of Form IV,
c) crystallising the (R)-Baclofen-cinnamic acid cocrystal Form IV.

In a second aspect, the present invention relates to a cocrystal of (R)-Baclofen with benzoic acid.

The molar ratio of (R)-Baclofen to benzoic acid in the above cocrystal is about 2:1.

The preferred embodiment of this aspect is the cocrystal of (R)-Baclofen with benzoic acid Form II, further: (R)-Baclofen-benzoic acid cocrystal Form II or cocrystal Form II, which is morphologically homogeneous, contaminant-free and stable form. In particular, it has high aqueous solubility and thermal stability, compared to prior art crystal forms A and B of (R)-Baclofen.

The (R)-Baclofen-benzoic acid cocrystal Form II of the present invention has an X-ray powder diffraction pattern with peaks at 2-theta angles of 3.4±0.2 degrees, 7.0±0.2 degrees, 10.9±0.2 degrees, 12.7±0.2 degrees, 16.0±0.2 degrees, 17.2±0.2 degrees, 19.2±0.2 degrees, 19.6±0.2 degrees, 20.0±0.2 degrees, 22.1±0.2 degrees, 23.7±0.2 degrees, 25.0±0.2 degrees and 28.5±0.2 degrees, when using Cu-Ka radiation. Further details regarding the X-ray powder diffraction measurement are provided herein below.

Furthermore, the cocrystal Form II of the present invention has an X-ray powder diffraction pattern with peaks and relative intensities as shown in Table 5.

**Table 5: XRPD of (R)-Baclofen-benzoic acid cocrystal Form II**

| **No.** | **2-theta (deg)** | **d-spacing [Å]** | **Relative intensity (%)** |
|---|---|---|---|
| 1 | 3.4 | 26.09 | 6 |
| 2 | 7.0 | 12.61 | 16 |
| 3 | 10.9 | 8.13 | 19 |
| 4 | 12.7 | 6.96 | 17 |
| 5 | 16.0 | 5.55 | 15 |
| 6 | 16.6 | 5.33 | 13 |
| 7 | 17.2 | 5.16 | 22 |
| 8 | 19.2 | 4.63 | 25 |
| 9 | 19.6 | 4.53 | 40 |
| 10 | 20.0 | 4.44 | 26 |
| 11 | 21.1 | 4.21 | 17 |
| 12 | 22.1 | 4.03 | 17 |
| 13 | 23.7 | 3.75 | 22 |
| 14 | 25.0 | 3.56 | 100 |
| 15 | 26.2 | 3.40 | 11 |
| 16 | 28.5 | 3.13 | 17 |

Furthermore, the cocrystal Form II of the present invention has a first endothermic peak with an onset at 103.9 ± 1 °C and a second endothermic peak with an onset at 153.4 ± 1 °C. Details of the differential scanning calorimetry measurement are provided further, in the experimental part.

Preferably, the cocrystal Form II as defined above is substantially chemically pure, i.e. contains less than 1 area-%, or less than 0.5 area-%, preferably less than 0.1 area-%, of compounds other than (R)-Baclofen and benzoic acid as determined by high performance liquid chromatography (HPLC).

Furthermore, the cocrystal Form II of the present invention is substantially polymorphically pure, and preferably contains less than 5 wt.-% of other crystalline, non-crystalline or amorphous forms of (R)-Baclofen, benzoic acid and cocrystals thereof.

The present invention relates also to a process for preparing (R)-Baclofen-benzoic acid cocrystal Form II as characterised above, wherein said process comprises the following steps:
a) contacting (R)-Baclofen and benzoic acid in a molar ratio of (R)-Baclofen to benzoic acid within a range 1:1-1:5 with a solvent selected from methyl isobutyl ketone, dioxane, cyclohexane, heptane, acetonitrile, ethyl acetate, methanol, ethanol, isopropanol, preferably with methyl isobutyl ketone,
b) optionally, seeding a mixture of step a) with pre-existing crystals of Form II,
c) crystallising the (*R*)-Baclofen-benzoic acid cocrystal Form II.

In a third aspect, the present invention relates to a cocrystal of (R)-Baclofen with salicylic acid.

The molar ratio of (R)-Baclofen to salicylic acid in the above cocrystal is about 1:1.

In the preferred embodiment of this aspect of the present invention, the cocrystal of (R)-Baclofen with salicylic acid Form VIII, further: (R)-Baclofen-salicylic acid cocrystal Form VIII or cocrystal Form VIII, is morphologically homogeneous, contaminant-free and stable form. In particular, it has high aqueous solubility and thermal stability, compared to prior art crystal forms A and B of (R)-Baclofen.

The (R)-Baclofen-salicylic acid cocrystal Form VIII of the present invention has an X-ray powder diffraction pattern with peaks at 2-theta angles of 5.5±0.2 degrees, 14.8±0.2 degrees, 16.5±0.2 degrees, 18.9±0.2 degrees, 19.7±0.2 degrees, 20.3±0.2 degrees, 20.5±0.2 degrees, 20.8±0.2 degrees, 21.4±0.2 degrees, 21.7±0.2 degrees, 25.1±0.2 degrees and 27.6±0.2 degrees, when using Cu-Ka radiation. Further details of the X-ray powder diffraction measurement are provided herein below, in the experimental part.

In more detail, the cocrystal Form VIII of the present invention has an X-ray powder diffraction pattern with peaks and/or relative intensities as shown in Table 6.

**Table 6: XRPD of (R)-Baclofen-salicylic acid cocrystal Form VIII**

| **No.** | **2-theta (deg)** | **d-spacing [Å]** | **Relative intensity (%)** |
|---|---|---|---|
| 1 | 3.7 | 24.17 | 3 |
| 2 | 5.5 | 16.14 | 100 |
| 3 | 10.9 | 8.14 | 6 |
| 4 | 12.3 | 7.21 | 8 |
| 5 | 14.8 | 6.00 | 7 |
| 6 | 16.5 | 5.38 | 14 |
| 7 | 18.5 | 4.80 | 7 |
| 8 | 18.9 | 4.69 | 11 |
| 9 | 19.7 | 4.50 | 29 |
| 10 | 20.3 | 4.38 | 44 |
| 11 | 20.5 | 4.33 | 18 |
| 12 | 20.8 | 4.26 | 12 |
| 13 | 21.4 | 4.15 | 14 |
| 14 | 21.7 | 4.09 | 18 |
| 15 | 23.3 | 3.81 | 6 |
| 16 | 24.7 | 3.60 | 9 |
| 17 | 25.1 | 3.55 | 10 |
| 18 | 26.0 | 3.42 | 5 |
| 19 | 27.0 | 3.30 | 5 |
| 20 | 27.6 | 3.23 | 12 |
| 21 | 28.3 | 3.15 | 5 |
| 22 | 29.4 | 3.04 | 6 |
| 23 | 30.0 | 2.98 | 2 |
| 24 | 31.0 | 2.89 | 3 |
| 25 | 32.3 | 2.77 | 2 |
| 26 | 33.6 | 2.67 | 2 |
| 27 | 35.3 | 2.54 | 3 |
| 28 | 37.4 | 2.40 | 3 |
| 29 | 38.5 | 2.34 | 2 |

Furthermore, the cocrystal Form VIII of the present invention has a first endothermic peak with an onset at 115.7 ± 1 °C followed by two overlapping endothermic peaks with onsets with onsets at 140,7 ± 1 °C and 142,7 ± 1 °C. Details of the differential scanning calorimetry measurement are provided further, in the experimental part.

Preferably, the cocrystal Form VIII of the present invention is substantially chemically pure, i.e. contains less than 1 area-%, or less than 0.5 area-%, preferably less than 0.1 area-%, of compounds other than (R)-Baclofen and salicylic acid as determined by high performance liquid chromatography (HPLC).

Furthermore, the cocrystal Form VIII of the present invention is substantially polymorphically pure, and preferably contains less than 5 wt.-% of other crystalline, non-crystalline or amorphous forms of (R)-Baclofen, salicylic acid and cocrystals thereof.

The present invention relates also to a process for preparing (R)-Baclofen-salicylic acid cocrystal Form VIII as characterised above, wherein said process comprises the following steps:
a) contacting (R)-Baclofen and salicylic acid in a molar ratio of (R)-Baclofen to salicylic acid within a range 1:1-1:5 with a solvent selected from methyl isobutyl ketone, dichloromethane, dioxane, toluene, dimethylformamide, heptane, tert-butyl methyl ether, tetrahydrofuran, acetonitrile, isopropanol, ethanol, methanol, acetone, ethyl acetate, preferably from tert-butyl methyl ether,
b) optionally, seeding a mixture of step a) with pre-existing crystals of Form VIII,
c) crystallising the (R)-Baclofen-salicylic acid cocrystal Form VIII.

The fourth aspect of the present invention relates to a cocrystal of (R)-Baclofen with ferulic acid.

The molar ratio of (R)-Baclofen to ferulic acid in the above cocrystal is about 1:1.

The preferred embodiment of this aspect of the invention relates to cocrystal of (R)-Baclofen with ferulic acid Form XII, further: (R)-Baclofen-ferulic acid cocrystal Form XII or cocrystal Form XII, is morphologically homogeneous, contaminant-free and stable form. In particular, it has high aqueous solubility and thermal stability, compared to prior art crystal forms A and B of (R)-Baclofen.

The (R)-Baclofen-ferulic acid cocrystal Form XII of the present invention has an X-ray powder diffraction pattern with peaks at 2-theta angles of 4.62±0.2 degrees, 7.48±0.2 degrees, 8.29±0.2 degrees, 14.56±0.2 degrees, 15.84±0.2 degrees, 17.26±0.2 degrees, 20.25±0.2 degrees, 23.05±0.2 degrees, 24.54±0.2 degrees, and 25.67±0.2 degrees, when using Cu-Ka radiation. Further details of the X-ray powder diffraction measurement are provided further, in the experimental part.

In more detail, the cocrystal Form XII of the present invention has an X-ray powder diffraction pattern with peaks and relative intensities as shown in Table 7.

**Table 7: XRPD of (R)-Baclofen-ferulic acid cocrystal Form XII**

| **No.** | **2-theta (deg)** | **d-spacing (Å)** | **Relative intensity (%)** |
|---|---|---|---|
| 1 | 4.622(2) | 19.104(9) | 5.95 |
| 2 | 6.41(4) | 13.78(9) | 0.13 |
| 3 | 7.480(4) | 11.809(6) | 3.80 |
| 4 | 8.299(2) | 10.645(3) | 7.28 |
| 5 | 12.491(10) | 7.081(6) | 1.76 |
| 6 | 12.824(8) | 6.898(4) | 2.16 |
| 7 | 14.563(4) | 6.0774(18) | 34.38 |
| 8 | 14.822(11) | 5.972(4) | 11.94 |
| 9 | 15.8425(19) | 5.5895(7) | 100.00 |
| 10 | 16.567(7) | 5.346(2) | 9.36 |
| 11 | 17.262(7) | 5.1330(19) | 12.74 |
| 12 | 18.072(8) | 4.905(2) | 6.15 |
| 13 | 18.319(19) | 4.839(5) | 6.26 |
| 14 | 18.824(5) | 4.7104(12) | 18.16 |
| 15 | 19.304(6) | 4.5942(13) | 16.85 |
| 16 | 19.671(12) | 4.509(3) | 33.07 |
| 17 | 19.851(7) | 4.4690(16) | 21.26 |
| 18 | 20.132(16) | 4.407(3) | 14.95 |
| 19 | 20.254(3) | 4.3810(7) | 89.40 |
| 20 | 21.267(16) | 4.174(3) | 1.78 |
| 21 | 22.183(13) | 4.004(2) | 7.49 |
| 22 | 22.61(5) | 3.930(8) | 13.41 |
| 23 | 23.051(4) | 3.8552(7) | 40.72 |
| 24 | 23.337(12) | 3.809(2) | 24.49 |
| 25 | 24.201(13) | 3.675(2) | 16.07 |
| 26 | 24.540(8) | 3.6246(12) | 24.09 |
| 27 | 25.196(8) | 3.5317(11) | 20.19 |
| 28 | 25.673(8) | 3.4672(10) | 25.91 |
| 29 | 26.41(2) | 3.373(3) | 1.80 |
| 30 | 27.26(2) | 3.269(3) | 17.77 |
| 31 | 27.734(4) | 3.2140(5) | 62.18 |
| 32 | 28.30(3) | 3.151(3) | 8.35 |
| 33 | 28.749(18) | 3.1028(19) | 2.53 |
| 34 | 30.08(2) | 2.968(2) | 7.84 |
| 35 | 30.43(2) | 2.935(2) | 5.06 |
| 36 | 30.71(3) | 2.909(2) | 3.26 |
| 37 | 31.09(3) | 2.874(3) | 4.69 |
| 38 | 31.47(2) | 2.840(2) | 4.46 |
| 39 | 31.915(7) | 2.8018(6) | 3.82 |
| 40 | 32.421(14) | 2.7593(11) | 2.56 |
| 41 | 33.813(15) | 2.6488(11) | 2.90 |
| 42 | 34.23(3) | 2.6177(19) | 3.18 |
| 43 | 34.56(2) | 2.5934(15) | 2.75 |
| 44 | 35.65(3) | 2.516(2) | 2.32 |

Furthermore, the cocrystal Form XII of the present invention, in a differential scanning calorimetry plot has three overlapping endothermic peaks with extrapolated onsets at 116.7± 1°C, 127.2± 1°C and 135.7± 1 °C. Further details of the differential scanning calorimetry measurement are provided in the experimental part.

Preferably, the cocrystal Form XII of the present invention is substantially chemically pure, i.e. it contains less than 1 area-%, or less than 0.5 area-%, preferably less than 0.1 area-%, of compounds other than (R)-Baclofen and ferulic acid as determined by high performance liquid chromatography (HPLC).

Furthermore, the cocrystal Form XII of the present invention is substantially polymorphically pure, and preferably contains less than 5 wt.-% of other crystalline, non-crystalline or amorphous forms of (R)-Baclofen, ferulic acid and cocrystals thereof.

The present invention relates also to a process for preparing (R)-Baclofen-ferulic acid cocrystal Form XII as characterised above, wherein said process comprises the following steps:
a) contacting (R)-Baclofen and ferulic acid in a molar ratio of (R)-Baclofen to ferulic acid within a range 1:1-1:5 with a solvent selected from a solvent selected from water, methanol, ethanol, isopropanol, acetone and a mixture thereof, preferably with acetone/water mixture,
b) optionally, seeding a mixture of step a) with pre-existing crystals of Form XII,
d) crystallising the (*R*)-Baclofen-ferulic acid cocrystal Form XII.

The present invention refers therefore in particular to any of the above characterised cocrystals of (R)-Baclofen with coformers. In particular, it relates to cocrystals of (R)-Baclofen with cinnamic acid (Forms III and IV), with benzoic acid (Form II), with salicylic acid (Form VIII) and/or with ferulic acid (Form XII).

Crystalline stability study of the new cocrystals found during the performed screening showed that under accelerated ICH storage conditions (40°C, 75±5 % RH) for 1 week and 4 weeks the (R)-Baclofen-cinnamic acid cocrystal Form III, (R)-Baclofen-cinnamic acid cocrystal Form IV, (R)-Baclofen-benzoic acid cocrystal Form II, (R)-Baclofen-salicylic acid cocrystal Form VIII and (R)-Baclofen-ferulic acid cocrystal Form XII remain stable after 4 weeks at 40°C/ 75 ± 5 % RH.

The term "cocrystal" as used herein, refers to a solid that is a crystalline single phase material composed of two or more, preferably two, different molecular or ionic compounds usually in a stoichiometric ratio, which is neither a solvate nor a simple salt. As to pharmaceutical ingredients, the term "cocrystal" refers to a crystalline form of a substance which comprises the drug component and at least one further component (coformer), e.g. (R)-Baclofen as the drug component and cinnamic acid as the coformer.

Further object of the invention is a pharmaceutical composition comprising a cocrystal of (R)-Baclofen with a coformer, preferably a cocrystal of (R)-Baclofen with the coformer selected from cinnamic acid, benzoic acid, salicylic acid and ferulic acid, and more preferably a cocrystal selected from the group consisting of (R)-Baclofen-cinnamic acid Form III, (R)-Baclofen-cinnamic acid Form IV, (R)-Baclofen-benzoic acid Form II, (R)-Baclofen-salicylic acid Form VIII and (R)-Baclofen-ferulic acid Form XII, and at least one pharmaceutically acceptable excipient.

In the preferred embodiment of the above, the pharmaceutical composition according to the present invention is formulated into a solid unit dosage form for oral administration, preferably into a tablet.

Exemplary pharmaceutically acceptable excipients which can be contemplated for use in the pharmaceutical composition of the present invention are described below.

Fillers are excipients which increase the volume of e.g. tablets. Examples of fillers are lactose, mannitol, celluloses such as microcrystalline cellulose, synthetic polymers, Ca-phosphate, inorganic calcium salts, maize starch, polyols and pregelatinized starch.

Binding agents are excipients which increase the adhesion of the active agents and excipients during granulation. Examples of binding agents are: sugars, such as sorbitol, glucose, fructose, disaccharides as saccharose, polysaccharides; acacia gum; cellulose derivatives, such as soluble celluloses like methylcellulose, hydroxypropyl methylcellulose and hydroxypropyl cellulose; tragacanth; polyvinylpyrrolidone, such as N-vinylpyrrolidone or polyvinylpyrrolidonevinyl acetate copolymer; sodium alginate and alginate derivatives and gelatin.

Disintegrants are excipients which can take up water and swell and thus improve disintegration of a tablet or granules. Examples of disintegrants are: croscarmellose sodium; starch (paste and pre-gelatinized), such as sodium starch glycolate, croscarmellose sodium and low substituted hydroxypropyl cellulose, methacrylic acid polymer with divinylbenzene, potassium salt, maltodextrin; and crospovidone.

Lubricants are excipients which reduce the friction between excipients and compression tooling surfaces. Examples of lubricants are magnesium stearate, magnesium fumarate, fumaric acid, and sodium stearyl fumarate.

A polymer, or a plurality of polymers may be used to coat the solid dosage form, if necessary, and they include cellulose derivatives, e.g. hydroxypropyl methylcellulose, polyvinylpyrrolidones, polyethylene glycols, polyvinyl alcohols, acrylates, such as polymethacrylate, cyclodextrins and copolymers and derivatives thereof, including for example polyvinylpyrolidine-vinylacetate. In some preferred embodiments the polymer or the plurality of polymers are pH dependent enteric polymers. Such polymers include cellulose derivatives, e.g. cellulose acetate phthalate, hydroxypropyl methyl cellulose phthalates, hydroxypropyl methyl acetate succinate, hydroxypropyl methyl cellulose acetate, carboxymethylcellulose or a salt thereof, e.g. the sodium salt, cellulose acetate trimellitate, hydroxypropyl cellulose acetate phthalate, or polymethyl acrylates, e.g. Eudragit®S.

Surfactants include but are not limited to, sorbitan fatty esters, polyoxymethylene sorbit esters, sodium lauryl sulfate, sodium docedylbenzenesulfate, dioctyl sodium sulfosuccinate, sodium stearate, EDTA or vitamin E or tocopherol derivatives.

Other excipients are known in the art and can be chosen by a skilled person depending on their function. Reference may be made in this context to Handbook of Pharmaceutical Excipients, ed. by R.C. Rowe et al, 6th edition, Pharmaceutical Press (2009).

Formulating the cocrystals into a pharmaceutical dosage form may be carried out by applying techniques and excipients known in the art. Reference is made herein e.g. to Pharmaceutical Manufacturing Handbook; Production and Processes, ed. S.C. Gad; John Wiley and Sons, (2008).

Another object of the present invention is the cocrystal of (R)-Baclofen with a coformer, in particular a cocrystal of (R)-Baclofen with the coformer selected from cinnamic acid, benzoic acid, salicylic acid and ferulic acid, and more preferably a cocrystal selected from the group consisting of (R)-Baclofen-cinnamic acid Form III, (R)-Baclofen-cinnamic acid Form IV, (R)-Baclofen-benzoic acid Form II, (R)-Baclofen-salicylic acid Form VIII and (R)-Baclofen-ferulic acid Form XII, or the pharmaceutical composition comprising any of the above cocrystals for the treatment of a disease or disorder selected from spasticity due to multiple sclerosis, spinal cord injury or cerebral palsy, and alcoholism.

### Experimental part

### Measuring instruments, conditions and protocols:

Within the meaning of the present invention, the term "room temperature" as used herein is understood to mean temperatures of about 15 °C to about 25 °C.

Herein, the X-ray powder diffraction peaks of the cocrystals are described by their 2theta angles with a tolerance of "±0.2". In the context of the present invention, it is also possible to use a smaller tolerance of "±0.1 ".

Regarding the synthesis of (R)-Baclofen, reference is made to examples 1 to 3 herein, as well as to example 1 and 4 of EP874804 B1. (R)-Baclofen as the starting material for preparing the cocrystals of the present invention can be prepared using any method known in the art. (R)-Baclofen hydrochloride (obtained in example 4) can also be used as a starting material for crystallizing the cocrystals of the present invention and can be prepared using any method known in the art.

### XRPD

| X-Ray | 40 kV, 15 mA | Scan speed / Duration time | 6.0000 deg/min |
|---|---|---|---|
| Goniometer | MiniFlex 300/600 | Step width | 0.0100 deg |
| Attachment | ASC-6 | Scan axis | Theta/2-Theta |
| Filter | K-beta(x1) | Scan range | 3.0000 - 36.0000 deg |
| CBO selection slit | - | Incident slit | 1.250deg |
| Diffrected beam mono. | None | Length limiting slit | 10.0mm |
| Detector | | Receiving slit #1 | 13.0mm |
| Scan mode | CONTINUOUS | Receiving slit #2 | 13.0mm |

### DSC

DSC analysis were carried out using DSC 2 Mettler Toledo Stare thermal analysis system at the temperature range of 25-300°C with the gradient of 10°C/min. Aluminum melting crucibles with the capacity of 40µl were used. Nitrogen was used as carrier gas with the flow of 50ml/min.

### ¹H-NMR

Proton nuclear magnetic resonance spectra were recorded in deuterium oxide (D₂O) using a Varian Mercury 400 MHz spectrometer equipped with a broadband probe ATB 1H/19F/X of 5 mm. Spectra were acquired for samples of 5-10 mg dissolved in 0.7ml of D₂O.

### HPLC

Sample concentration : 5mg/5ml in ACN/H2O-0.1 methane sulfonic acid 20:80
Injection Volume: 10 µl;
Run Time: 20.0 minutes;
Column: PRIMASEP100, 4.6x150 mm;100A,5µm;
Mobile phase: gradient: ACN/H2O- 0.1 methane sulfonic acid 20:80 / ACN (95:5) -(75:25) - (95:5)
Column temp.: 40°C; and
Flow rate: 1 ml/min.

### Aqueous solubility studies

Solubility tests were performed according to 2018 edition of USP, General Notices, point 5.30. Description and Solubility.

The approximate solubility of a compendial substance is indicated by one of the following descriptive terms.

| Descriptive Term | Parts of solvent required for 1 part of solute |
|---|---|
| Very soluble | Less than 1 |
| Freely soluble | From 1 to 10 |
| Soluble | From 10 to 30 |
| Sparingly soluble | From 30 to 100 |
| Slightly soluble | From 100 to 1000 |
| Very slightly soluble | From 1000 to 10000 |
| Practically insoluble or insoluble | Greater than or equal to 10000 |

### Crystalline stability test

A test for stability against humidity was performed as follows: The sample is exposed to a relative humidity of 75% (+/- 5%) at 40°C (+/- 2°C) for 4 weeks. The expression "crystalline stability" means that no conversion into another crystalline form occurs, as determined by XRPD.

The equilibrium relative humidity of a sample is measured by determining the relative humidity in % in the air above a test sample, after establishment of a humidity equilibrium in a closed/open system at a constant temperature.

The following examples describe the present invention in detail, but are not to be construed to limit the present invention.

### Example 1 -Preparation of diastereoisomeric salt of (R)-isomer of 3-(4-chlorophenyl)-qlutaramide and (S)-(-)-α-methylbenzylamine

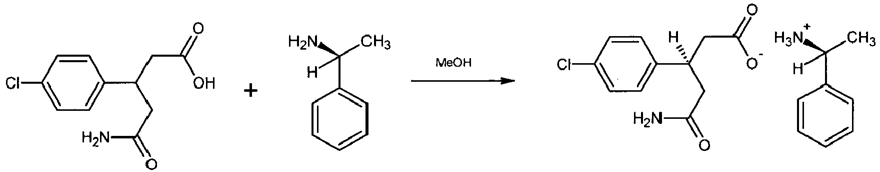

The reactor was charged with racemic 3-(4-chlorophenyl)-glutaramide ((R,S)-GAM) (175g), methanol (1375ml) and the resulting reaction mass was heated at 60-65 °C to dissolve ((R,S)-GAM). (S)-(-)-α-Methylbenzylamine (S-MBA) (94.5 ml) was added dropwise with stirring and the resulting 1:1 GAM:MBA solution was cooled slowly to ambient temperature (20-25°C). The crystalline GAM:MBA salt was filtered off, washed with methanol (3x33ml), and dried at 40-50°C to afford white crystals (R-GAM: S-MBA salt). Yield: 104 g (40%).

### Example 2 -Preparation of diastereoisomeric salt of (3R)-(4-chlorophenyl)-glutaramide

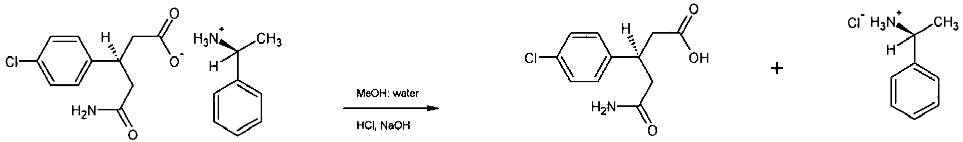

Diastereoisomeric salt of (R)-isomer of 3-(4-chlorophenyl)-glutaramide and (S)-(-)-α-methylbenzylamine (R-GAM:S-MBA) (100 g) was dissolved in 2,5% sodium hydroxide aqueous solution (670 ml) at 20-25 °C. Methanol (670 ml) was added thereto. Then concentrated hydrochloric acid was slowly added to adjust the pH to 2.3. During addition, (3R)-(4-chlorophenyl)-glutaramide precipitated. The mixture was allowed to stand for 1 hour, and then the precipitated solid was separated by filtration to obtain (3R)-(4-chlorophenyl)-glutaramide (R-GAM). Yield: 46.5 g (70%).

### Example 3 -Preparation of (R)-Baclofen

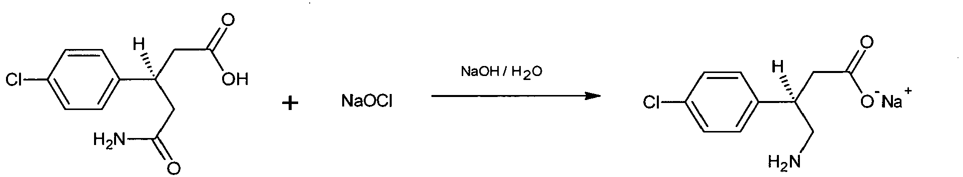

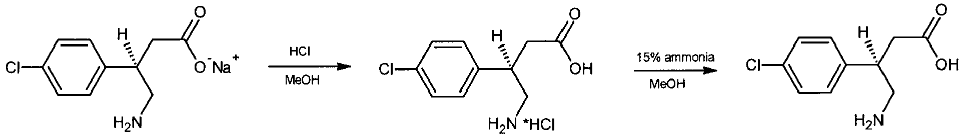

(3R)-(4-chlorophenyl)-glutaramide (R-GAM) (36 g) was dissolved in aqueous solution of NaOH (20g/200ml). Then sodium hypochlorite (108 g) was added at 0-5 °C and stirred for 6h at 35-40 °C. After completion of Hoffman rearrangement, methanol (326 ml) was added. pH was adjusted to 2.2-2.8 by addition of concentrated hydrochloric acid, and then activated carbon was added. Reaction was stirred for 1h and activated carbon was filtered off through celite bed. The product was isolated by pH adjustment to 6.5-7.0 with 15% ammonia solution and the resultant precipitate was filtered off and dried at 60°C to afford (R)-Baclofen.
Yield: 19,3 g (62%). Purity: 99.9 % by HPLC.

### Example 4 - Preparation of (R)-Baclofen hydrochloride

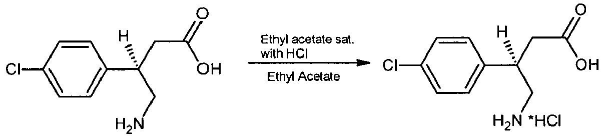

The reactor was charged with (R)-Baclofen (20 g) and ethyl acetate (250 ml). Ethyl acetate saturated with hydrochloric acid was added (32.5 ml) to the reaction mass and the resulting mixture was stirred for 1h. The precipitate was filtered off and dried at 50-60°C to afford (R)-Baclofen hydrochloride.

Yield: 15.4 g (79%). Purity: 99.9% by HPLC.

### Examples of cocrystallization of (R)-Baclofen with cinnamic acid to obtain cocrvstal Form III Example 5

A round bottom flask equipped with magnetic stirrer was charged with (R)-Baclofen (2.0 g, 9.4 mmol), cinnamic acid (2.77 g, 18.7 mmol, 2 eq) and ethyl acetate (20 ml) and the reaction mixture was stirred for 15 hrs at room temperature. Obtained solid was filtered through sinter funnel and washed with ethyl acetate (3x2ml). The product was dried under vacuum at room temperature and (R)-Baclofen-cinnamic acid cocrystal Form III (as confirmed by XRPD and DSC) with a purity of above 99.90% (78% yield) was obtained.

### Example 6

A round bottom flask equipped with magnetic stirrer was charged with (R)-Baclofen (2.0 g, 9.4 mmol), cinnamic acid (2.77 g, 18.7 mmol, 2 eq) and ethyl acetate (20 ml). The resulting suspension was seeded with (R)-Baclofen-cinnamic acid cocrystal Form III and stirred for 15 hrs at room temperature. Obtained solid was filtered through sinter funnel and washed with ethyl acetate (3x2ml). The product was dried under vacuum at room temperature and (R)-Baclofen-cinnamic acid cocrystal Form III (as confirmed by XRPD and DSC) with a purity of above 99.90% (82% yield) was obtained.

### Example 7

A round bottom flask equipped with magnetic stirrer was charged with (R)-Baclofen hydrochloride (2.0 g, 7.9 mmol) and methanol: water mixture (20ml) (75:25), then pH was adjusted to 6,5 - 7,0 using 15% ammonia. The obtained product was filtered off and charged into another reactor containing ethyl acetate (20 ml) and cinnamic acid (1.88g). The resulting suspension was seeded with (R)-Baclofen-cinnamic acid cocrystal Form III and stirred for 15 hrs at room temperature. Obtained solid was filtered through sinter funnel and washed with ethyl acetate (3x2ml). The product was dried under vacuum at room temperature and (R)-Baclofen-cinnamic acid cocrystal Form III (as confirmed by XRPD and DSC) with a purity of above 99.90% (78% yield) was obtained.

### Example 8 - Characterization of (R)-Baclofen-cinnamic acid cocrystal Form III

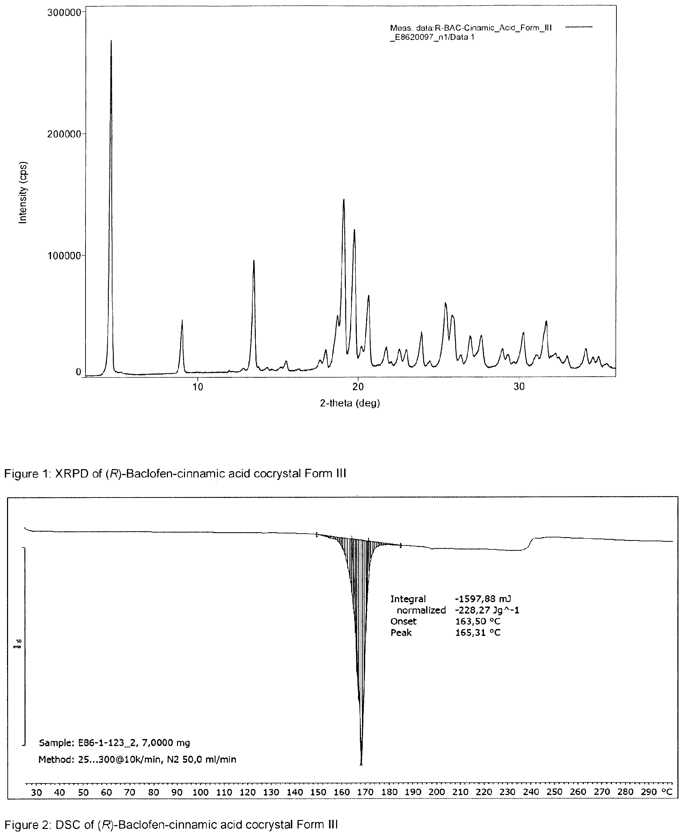

Crystalline (R)-Baclofen-cinnamic acid cocrystal Form III can be characterized by a X-ray powder diffraction pattern having peaks at 2-theta angles of 4.60, 9.03, 13.51, 15.51,17.99, 18.70, 19.11, 19.77, 20.65 and 23.90 degrees ± 0.2 degrees, in particular an X-ray powder diffraction pattern as shown in Figure 1.

(R)-Baclofen-cinnamic acid cocrystal Form III may be also characterized by thermal analysis. A representative differential scanning calorimetry (DSC) plot for (R)-Baclofen-cinnamic acid cocrystal Form III is shown in Figure 2. The DSC plot comprises an endothermic peak with an onset temperature of 163.5± 1°C.

Figure 1 shows a representative XRPD pattern of (R)-Baclofen-cinnamic acid cocrystal Form III, wherein the numerical data are summarized in Table 8:

**Table 8: XRPD of (R)-Baclofen-cinnamic acid cocrystal Form III**

| **No.** | **2-theta (deg)** | **d-spacing (Å)** | **Relative intensity (%)** |
|---|---|---|---|
| 1 | 4.6034(8) | 19.180(3) | 100.00 |
| 2 | 5.41(4) | 16.31(13) | 1.85 |
| 3 | 9.0380(16) | 9.7766(17) | 17.52 |
| 4 | 11.969(8) | 7.388(5) | 0.26 |
| 5 | 12.854(19) | 6.882(10) | 0.65 |
| 6 | 13.5154(14) | 6.5462(7) | 41.46 |
| 7 | 13.788(12) | 6.418(6) | 0.54 |
| 8 | 14.335(13) | 6.174(6) | 1.27 |
| 9 | 14.63(4) | 6.052(16) | 0.49 |
| 10 | 15.167(17) | 5.837(7) | 1.42 |
| 11 | 15.519(6) | 5.705(2) | 3.52 |
| 12 | 17.646(11) | 5.022(3) | 3.45 |
| 13 | 17.999(6) | 4.9242(16) | 6.51 |
| 14 | 18.706(7) | 4.7399(17) | 49.92 |
| 15 | 19.1137(16) | 4.6396(4) | 52.96 |
| 16 | 19.7713(17) | 4.4867(4) | 60.34 |
| 17 | 20.211(9) | 4.3902(19) | 9.49 |
| 18 | 20.651(3) | 4.2975(6) | 30.02 |
| 19 | 21.728(8) | 4.0869(14) | 7.06 |
| 20 | 22.047(7) | 4.0285(13) | 0.92 |
| 21 | 22.546(8) | 3.9404(14) | 6.13 |
| 22 | 22.991(8) | 3.8652(14) | 5.65 |
| 23 | 23.903(8) | 3.7198(12) | 11.82 |
| 24 | 24.415(13) | 3.6429(19) | 0.76 |
| 25 | 25.415(7) | 3.5017(9) | 33.28 |
| 26 | 25.772(16) | 3.454(2) | 22.93 |
| 27 | 25.945(17) | 3.431(2) | 5.21 |
| 28 | 26.35(2) | 3.379(3) | 4.72 |
| 29 | 26.922(9) | 3.3091(11) | 13.95 |
| 30 | 27.648(10) | 3.2238(11) | 17.46 |
| 31 | 28.93(2) | 3.084(2) | 6.08 |
| 32 | 29.27(3) | 3.049(3) | 2.99 |
| 33 | 30.246(11) | 2.9525(10) | 13.39 |
| 34 | 31.01(7) | 2.881(6) | 5.54 |
| 35 | 31.686(11) | 2.8216(10) | 17.06 |
| 36 | 32.23(4) | 2.775(3) | 2.12 |
| 37 | 32.43(9) | 2.759(8) | 5.18 |
| 38 | 32.951(18) | 2.7161(15) | 1.79 |
| 39 | 34.14(2) | 2.6245(17) | 6.57 |
| 40 | 34.59(5) | 2.591(4) | 3.31 |
| 41 | 34.92(4) | 2.568(3) | 3.28 |
| 42 | 35.40(9) | 2.533(6) | 1.09 |

### Examples for cocrystallisation of (R)-Baclofen with cinnamic acid to obtain Form IV

### Example 9

A round bottom flask equipped with magnetic stirrer was charged with (R)-Baclofen (2.0 g, 9.4 mmol), cinnamic acid (2.77 g, 18.7 mmol, 2 eq) and acetone (20 ml) and the mixture was stirred for 15 hrs at room temperature. Obtained solid was filtered through sinter funnel and washed with ethyl acetate (3x2ml). The product was dried under vacuum at room temperature and (R)-Baclofen-cinnamic acid cocrystal Form IV (as confirmed by XRPD and DSC) with a purity of above 99.90% (92% yield) was obtained.

### Example 11

A round bottom flask equipped with magnetic stirrer was charged with (R)-Baclofen (2.0 g, 9.4 mmol), cinnamic acid (2.77 g, 18.7 mmol, 2 eq) and acetone (20 ml). The resulting suspension was seeded with (R)-Baclofen-cinnamic acid cocrystal Form IV and stirred for 15 hrs at room temperature. Obtained solid was filtered through sinter funnel and washed with ethyl acetate (3x2ml). The product was dried under vacuum at room temperature and (R)-Baclofen-cinnamic acid cocrystal Form IV (as confirmed by XRPD and DSC) with a purity of above 99.90% (95% yield) was obtained.

### Example 12

A round bottom flask equipped with magnetic stirrer was charged with (R)-Baclofen hydrochloride (2.0 g, 7.9 mmol) and methanol: water mixture (20ml) (75:25); pH was adjusted to 6,5-7,0 with 15% ammonia. The obtained product was filtered off and charged into another reactor containing acetone (20 ml) and cinnamic acid (1.88g). The resulting suspension was seeded with (R)-Baclofen-cinnamic acid cocrystal Form IV and stirred for 15 hrs at room temperature. Obtained solid was filtered through sinter funnel and washed with acetone (3x2ml). The solid product was dried under vacuum at room temperature and (R)-Baclofen-cinnamic acid cocrystal Form IV (as confirmed by XRPD and DSC) with a purity of above 99.90% (92% yield) was obtained.

### Example 13 - Characterization of (R)-Baclofen-cinnamic acid cocrystal Form IV

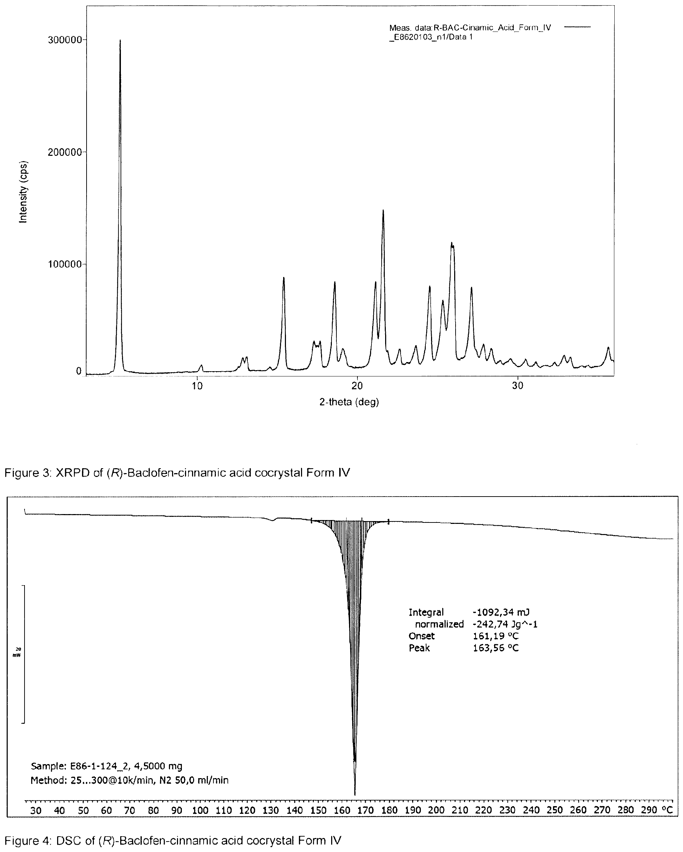

Crystalline (R)-Baclofen-cinnamic acid cocrystal Form IV can be characterized by a X-ray powder diffraction pattern having peaks at 2-theta angles of 5.15, 10.27, 12.84, 13.10, 15.42, 18.58, 21.14, 24.53, 25.34, 27.14 degrees ± 0.2 degrees, in particular an X-ray powder diffraction pattern as shown in Figure 3.

Crystalline (R)-Baclofen-cinnamic acid cocrystal Form IV may be also characterized by thermal analysis. A representative DSC plot for (R)-Baclofen-cinnamic acid cocrystal Form IV is shown in Figure 4. The DSC plot comprises an endothermic peak with an onset temperature of 161.2 ± 1°C.

Figure 3 shows a representative XRPD pattern of (R)-Baclofen-cinnamic acid cocrystal Form IV, wherein the numerical data are summarized in Table 9:

**Table 9 : XRPD of (R)-Baclofen-cinnamic acid cocrystal Form IV**

| **No.** | **2-theta (deg)** | **d-spacing (Å)** | **Realtive intensity (%)** |
|---|---|---|---|
| 1 | 5.1508(19) | 17.143(6) | 100.00 |
| 2 | 10.279(5) | 8.599(4) | 2.48 |
| 3 | 12.843(5) | 6.887(3) | 5.48 |
| 4 | 13.102(6) | 6.752(3) | 6.23 |
| 5 | 14.33(12) | 6.18(5) | 0.53 |
| 6 | 15.428(2) | 5.7388(8) | 36.97 |
| 7 | 17.28(2) | 5.128(6) | 12.42 |
| 8 | 17.673(6) | 5.0144(16) | 10.75 |
| 9 | 18.582(2) | 4.7711(6) | 37.61 |
| 10 | 19.09(3) | 4.646(6) | 8.77 |
| 11 | 19.25(2) | 4.606(5) | 2.61 |
| 12 | 21.142(3) | 4.1989(5) | 41.21 |
| 13 | 21.6200(16) | 4.1071(3) | 62.73 |
| 14 | 21.901(6) | 4.0550(10) | 2.38 |
| 15 | 22.630(7) | 3.9261(12) | 5.53 |
| 16 | 23.660(9) | 3.7573(14) | 6.66 |
| 17 | 24.531(3) | 3.6258(4) | 30.95 |
| 18 | 25.345(4) | 3.5113(6) | 25.74 |
| 19 | 25.830(4) | 3.4464(6) | 29.61 |
| 20 | 25.996(4) | 3.4248(5) | 46.33 |
| 21 | 27.140(3) | 3.2829(4) | 27.69 |
| 22 | 27.363(16) | 3.2568(18) | 6.38 |
| 23 | 27.872(8) | 3.1984(9) | 6.64 |
| 24 | 28.346(8) | 3.1460(9) | 4.89 |
| 25 | 28.87(2) | 3.090(2) | 0.51 |
| 26 | 29.54(2) | 3.021(2) | 2.63 |
| 27 | 30.48(2) | 2.9303(18) | 1.94 |
| 28 | 31.10(2) | 2.873(2) | 1.02 |
| 29 | 32.283(14) | 2.7707(11) | 1.24 |
| 30 | 32.880(8) | 2.7218(6) | 5.14 |
| 31 | 33.287(7) | 2.6894(6) | 4.94 |
| 32 | 33.96(4) | 2.638(3) | 0.52 |
| 33 | 34.350(9) | 2.6086(6) | 0.47 |
| 34 | 35.659(7) | 2.5158(5) | 6.18 |

### Examples for cocrystallisation of (R)-Baclofen with benzoic acid to obtain Form II

### Example 14

A round bottom flask equipped with magnetic stirrer was charged with (R)-Baclofen (300 mg, 1.4 mmol), benzoic acid (857 mg, 7.02 mmol, 5 eq) and methyl isobutyl ketone (3 ml) and the mixture was stirred for 15 hrs at room temperature. Obtained solid was filtered through sinter funnel and washed with methyl isobutyl ketone (3x1ml). The product was dried under vacuum at room temperature and (R)-Baclofen-benzoic acid cocrystal Form II (as confirmed by XRPD and DSC) with a purity of above 99.90% (78% yield) was obtained.

### Example 15

A round bottom flask equipped with magnetic stirrer was charged with (R)-Baclofen (300 mg, 1.4 mmol), benzoic acid (857 mg, 7.02 mmol, 5 eq) and methyl isobutyl ketone (3 ml). The resulting suspension was seeded with (R)-Baclofen-benzoic acid cocrystal Form II and stirred for 15 hrs at room temperature. Obtained solid was filtered through sinter funnel and washed with methyl isobutyl ketone (3x1ml). The product was dried under vacuum at room temperature and (R)-Baclofen-benzoic acid cocrystal Form II (as confirmed by XRPD and DSC) with a purity of above 99.90% (90% yield) was obtained.

### Example 16

A round bottom flask equipped with magnetic stirrer was charged with (R)-Baclofen hydrochloride (300 mg, 1.19 mmol) and methanol: water mixture (2ml) (75:25); pH was adjusted to 6,5-7,0 with 15% ammonia. The obtained product was filtered off and charged into another reactor containing methyl isobutyl ketone (3 ml) and benzoic acid (732.36 mg). The resulting suspension was seeded with (R)-Baclofen-benzoic acid cocrystal Form II and stirred for 15 hrs at room temperature. Obtained solid was filtered through sinter funnel and washed with methyl isobutyl ketone (3x1ml). The product was dried under vacuum at room temperature and (R)-Baclofen-benzoic acid cocrystal Form II (as confirmed by XRPD and DSC) with a purity of above 99.90% (90% yield) was obtained.

### Example 17 - Characterization of (R)-Baclofen-benzoic acid cocrystal Form II

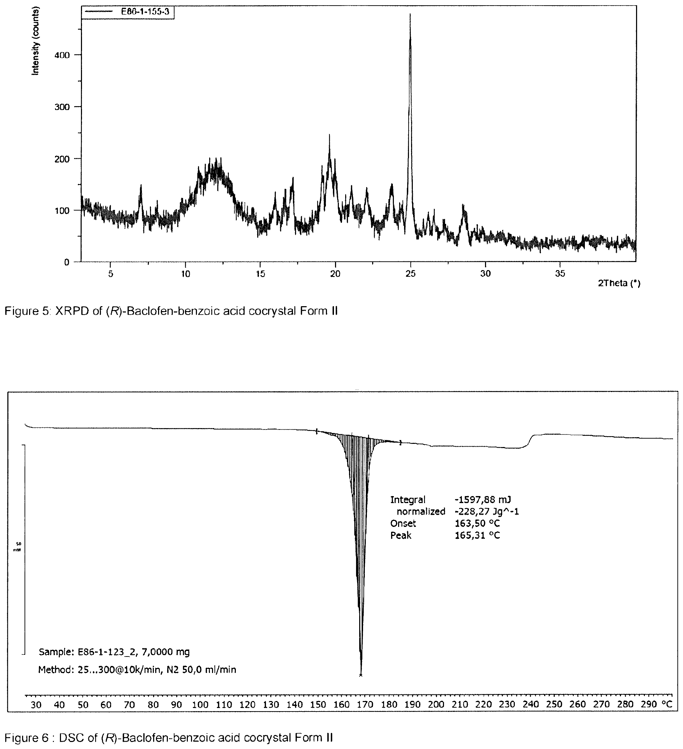

Crystalline (R)-Baclofen-benzoic acid cocrystal Form II can be characterized by an X-ray powder diffraction pattern having peaks at 2-theta angles of 3.4, 7.0, 10.9, 12.7, 16.0, 17.2, 19.2, 19.6, 20.0, 22.1, 23.7, 25.0, 28.5 degrees ± 0.2 degrees, in particular by an X-ray powder diffraction pattern as shown in Figure 5.

Crystalline (R)-Baclofen-benzoic acid cocrystal Form II may be also characterized by thermal analysis. A representative DSC plot for (R)-Baclofen-benzoic acid cocrystal Form II is shown in Figure 6. The DSC plot has a first endothermic peak with an onset at 103.9 ± 1 °C and a second endothermic peak with an onset at 153.4 ± 1 °C.

Figure 5 shows a representative XRPD pattern of (R)-Baclofen-benzoic acid cocrystal Form II, wherein the numerical data are summarized in Table 10:

**Table 10: XRPD of (R)-Baclofen-benzoic acid cocrystal Form II**

| **No.** | **2-theta(deg)** | **d-spacing [Å]** | **Relative intensity (%)** |
|---|---|---|---|
| 1 | 3.4 | 26.09 | 6 |
| 2 | 7.0 | 12.61 | 16 |
| 3 | 10.9 | 8.13 | 19 |
| 4 | 12.7 | 6.96 | 17 |
| 5 | 16.0 | 5.55 | 15 |
| 6 | 16.6 | 5.33 | 13 |
| 7 | 17.2 | 5.16 | 22 |
| 8 | 19.2 | 4.63 | 25 |
| 9 | 19.6 | 4.53 | 40 |
| 10 | 20.0 | 4.44 | 26 |
| 11 | 21.1 | 4.21 | 17 |
| 12 | 22.1 | 4.03 | 17 |
| 13 | 23.7 | 3.75 | 22 |
| 14 | 25.0 | 3.56 | 100 |
| 15 | 26.2 | 3.40 | 11 |
| 16 | 28.5 | 3.13 | 17 |

### Examples for cocrystallisation of (R)-Baclofen with salicylic acid to obtain Form VIII

### Example 18

A round bottom flask equipped with magnetic stirrer was charged with (R)-Baclofen (300 mg, 1.4 mmol) and salicylic acid (582 g, 4.21 mmol, 3 eq), tert-butyl methyl ether (3ml) and the mixture was stirred for 15 hrs at room temperature. Obtained solid was filtered through sinter funnel and washed with tert-butyl methyl ether (4x 0.3ml). The product was dried under vacuum at room temperature and (R)-Baclofen-salicylic acid cocrystal Form VIII (as confirmed by XRPD and DSC) with a purity of above 99.90% (80% yield) was obtained.

### Example 19

A round bottom flask equipped with magnetic stirrer was charged with (R)-Baclofen (300 mg, 1.4 mmol), salicylic acid (582 mg, 4.21 mmol, 3 eq) and tert-butyl methyl ether (3 ml). The resulting suspension was seeded with (R)-Baclofen-salicylic acid cocrystal Form VIII and stirred for 15 hrs at room temperature. Obtained solid was filtered through sinter funnel and washed with tert-butyl methyl ether (4x 0.3ml). The product was dried under vacuum at room temperature and (R)-Baclofen-salicylic acid cocrystal Form VIII (as confirmed by XRPD and DSC) with a purity of above 99.90% (85% yield) was obtained.

### Example 20

A round bottom flask equipped with magnetic stirrer was charged with (R)-Baclofen hydrochloride (300 mg, 1.19 mmol) and methanol: water mixture (3ml) (75:25); pH was adjusted to 6,5-7,0 with 15% ammonia. The obtained product was filtered off and charged into another reactor containing tert-butyl methyl ether (3 ml) and salicylic acid (3.54 g). The resulting suspension was seeded with (R)-Baclofen-salicylic acid cocrystal Form VIII and stirred for 15 hrs at room temperature. Obtained solid was filtered through sinter funnel and washed with tert-butyl methyl ether (4x 0.3ml). The product was dried under vacuum at room temperature and (R)-Baclofen-salicylic acid cocrystal Form VIII (as confirmed by XRPD and DSC) with a purity of above 99.90% (82% yield) was obtained.

### Example 21 - Characterization of (R)-Baclofen-salicylic acid cocrystal Form VIII

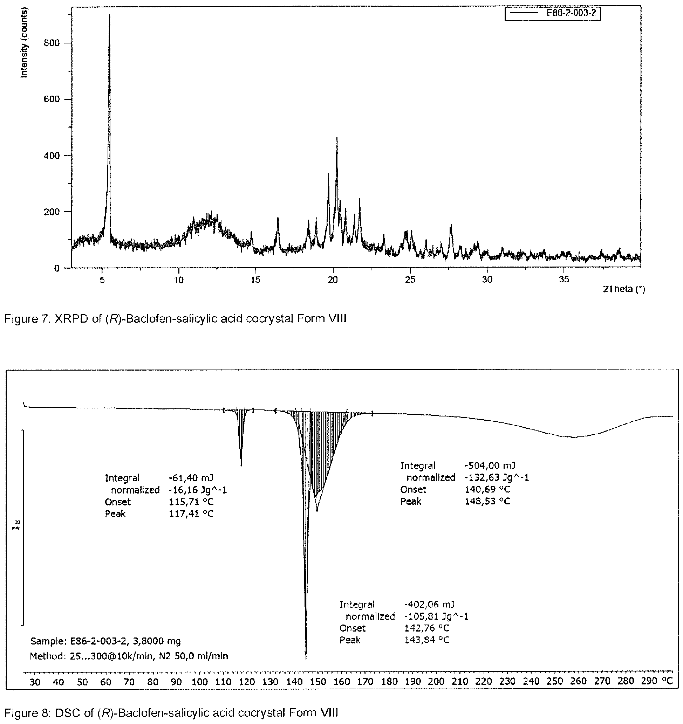

Crystalline (R)-Baclofen-salicylic acid cocrystal Form VIII can be characterized by an X-ray powder diffraction pattern having peaks at 2-theta angles of 5.5, 14.8, 16.5, 18.9, 19.7, 20.3, 20.5, 20.8, 21.4, 21.7, 25.1, 27.6 degrees ± 0.2 degree, in particular by an X-ray powder diffraction pattern as shown in Figure 7.

Crystalline (R)-Baclofen- salicylic acid cocrystal Form VIII may be also characterized by thermal analysis. A representative DSC plot for (R)-Baclofen-salicylic acid cocrystal Form VIII is shown in Figure 8. The DSC plot has a first endothermic peak with an onset at 115.7 ± 1 °C followed by two overlapped endothermic peaks with onset close to 140 ± 1 °C.

Figure 7 shows a representative XRPD pattern of (R)-Baclofen-salicylic acid cocrystal Form VIII, wherein the numerical data are summarized in Table 11:

**Table 11: XRPD of (R)-Baclofen-salicylic acid cocrystal Form VIII**

| **No.** | **2-theta(deg)** | **d-spacing [Å]** | **Relative intensity (%)** |
|---|---|---|---|
| 1 | 3.7 | 24.17 | 3 |
| 2 | 5.5 | 16.14 | 100 |
| 3 | 10.9 | 8.14 | 6 |
| 4 | 12.3 | 7.21 | 8 |
| 5 | 14.8 | 6.00 | 7 |
| 6 | 16.5 | 5.38 | 14 |
| 7 | 18.5 | 4.80 | 7 |
| 8 | 18.9 | 4.69 | 11 |
| 9 | 19.7 | 4.50 | 29 |
| 10 | 20.3 | 4.38 | 44 |
| 11 | 20.5 | 4.33 | 18 |
| 12 | 20.8 | 4.26 | 12 |
| 13 | 21.4 | 4.15 | 14 |
| 14 | 21.7 | 4.09 | 18 |
| 15 | 23.3 | 3.81 | 6 |
| 16 | 24.7 | 3.60 | 9 |
| 17 | 25.1 | 3.55 | 10 |
| 18 | 26.0 | 3.42 | 5 |
| 19 | 27.0 | 3.30 | 5 |
| 20 | 27.6 | 3.23 | 12 |
| 21 | 28.3 | 3.15 | 5 |
| 22 | 29.4 | 3.04 | 6 |
| 23 | 30.0 | 2.98 | 2 |
| 24 | 31.0 | 2.89 | 3 |
| 25 | 32.3 | 2.77 | 2 |
| 26 | 33.6 | 2.67 | 2 |
| 27 | 35.3 | 2.54 | 3 |
| 28 | 37.4 | 2.40 | 3 |
| 29 | 38.5 | 2.34 | 2 |

### Examples related to the cocrvstallization of (R)-Baclofen with ferulic acid to obtain Form XII

### Example 22

A round bottom flask equipped with magnetic stirrer was charged with (R)-Baclofen (2.0 g, 9.4 mmol), ferulic acid (5.45 g, 28.1 mmol, 3 eq) and acetone/water mixture (8:2) (20 ml) and the mixture was stirred for 15 hrs at room temperature. Obtained solid was filtered through sinter funnel and washed with acetone (3×4ml). The product was dried under vacuum at room temperature and (R)-Baclofen-ferulic acid cocrystal Form XII (as confirmed by XRPD and DSC) with a purity of above 99.88% (78% yield) was obtained.

### Example 23

A round bottom flask equipped with magnetic stirrer was charged with (R)-Baclofen (2.0 g, 9.4 mmol), ferulic acid (5.45 g, 28.1 mmol, 3 eq) and acetone: water mixture (8:2) (20 ml). The resulting suspension was seeded with (R)-Baclofen-ferulic acid cocrystal Form XII and stirred for 15 hrs at room temperature. Obtained solid was filtered through sinter funnel and washed with acetone (3×4ml). The product was dried under vacuum at room temperature and (R)-Baclofen-ferulic acid cocrystal Form XII (as confirmed by XRPD and DSC) with a purity of above 99.80% (95% yield) was obtained.

### Example 24

A round bottom flask equipped with magnetic stirrer was charged with (R)-Baclofen hydrochloride (2.0 g, 7.9 mmol) and methanol: water mixture (20ml) (75:25); pH was adjusted to 6,5-7,0 with 15% ammonia. The obtained product was filtered and charged into another reactor containing acetone: water mixture (20 ml) (80:20) and ferulic acid (3.54 g). The resulting suspension was seeded with (R)-Baclofen-ferulic acid cocrystal Form XII and stirred for 15 hrs at room temperature. Obtained solid was filtered through sinter funnel and washed with acetone (3×4ml). The product was dried under vacuum at room temperature and (R)-Baclofen-ferulic acid cocrystal Form XII (as confirmed by XRPD and DSC) with a purity of above 99.80% (92% yield) was obtained.

### Example 25 - Characterization of (R)-Baclofen-ferulic acid cocrystal Form XII

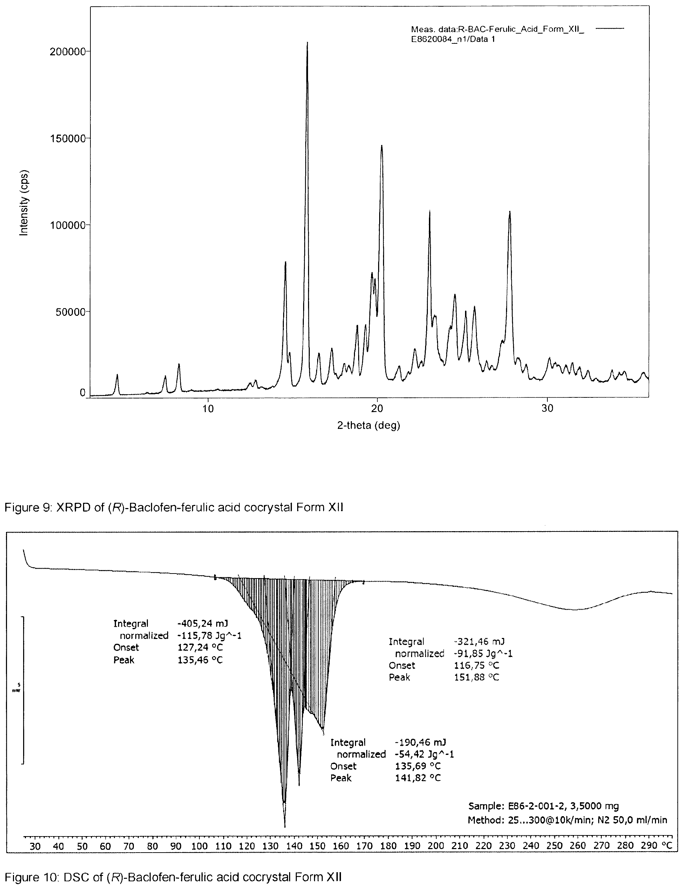

Crystalline (R)-Baclofen-ferulic acid cocrystal Form XII can be characterized by an X-ray powder diffraction pattern having peaks at 2-theta angles of 4.62, 7.48, 8.29, 14.56, 15.84, 17.26, 20.25, 23.05, 24.54, 25.67 degrees ± 0.2 degree, in particular by an X-ray powder diffraction pattern as shown in Figure 9.

Crystalline (R)-Baclofen-ferulic acid cocrystal Form XII may be also characterized by thermal analysis. A representative DSC plot for (R)-Baclofen-ferulic acid cocrystal Form XII is shown in Figure 10. The DSC plot comprises 3 overlapping endothermic peaks with extrapolated onsets between 116.7± 1°C and 135.7± 1 °C.

Figure 9 shows a representative XRPD pattern of (R)-Baclofen-ferulic acid cocrystal Form XII, wherein the numerical data are summarized in Table 12:

**Table 12: XRPD of (R)-Baclofen-ferulic acid cocrystal Form XII**

| **No.** | **2-theta (deg)** | **d-spacing (Å)** | **Relative intensity (%)** |
|---|---|---|---|
| 1 | 4.622(2) | 19.104(9) | 5.95 |
| 2 | 6.41(4) | 13.78(9) | 0.13 |
| 3 | 7.480(4) | 11.809(6) | 3.80 |
| 4 | 8.299(2) | 10.645(3) | 7.28 |
| 5 | 12.491(10) | 7.081(6) | 1.76 |
| 6 | 12.824(8) | 6.898(4) | 2.16 |
| 7 | 14.563(4) | 6.0774(18) | 34.38 |
| 8 | 14.822(11) | 5.972(4) | 11.94 |
| 9 | 15.8425(19) | 5.5895(7) | 100.00 |
| 10 | 16.567(7) | 5.346(2) | 9.36 |
| 11 | 17.262(7) | 5.1330(19) | 12.74 |
| 12 | 18.072(8) | 4.905(2) | 6.15 |
| 13 | 18.319(19) | 4.839(5) | 6.26 |
| 14 | 18.824(5) | 4.7104(12) | 18.16 |
| 15 | 19.304(6) | 4.5942(13) | 16.85 |
| 16 | 19.671(12) | 4.509(3) | 33.07 |
| 17 | 19.851(7) | 4.4690(16) | 21.26 |
| 18 | 20.132(16) | 4.407(3) | 14.95 |
| 19 | 20.254(3) | 4.3810(7) | 89.40 |
| 20 | 21.267(16) | 4.174(3) | 1.78 |
| 21 | 22.183(13) | 4.004(2) | 7.49 |
| 22 | 22.61(5) | 3.930(8) | 13.41 |
| 23 | 23.051(4) | 3.8552(7) | 40.72 |
| 24 | 23.337(12) | 3.809(2) | 24.49 |
| 25 | 24.201(13) | 3.675(2) | 16.07 |
| 26 | 24.540(8) | 3.6246(12) | 24.09 |
| 27 | 25.196(8) | 3.5317(11) | 20.19 |
| 28 | 25.673(8) | 3.4672(10) | 25.91 |
| 29 | 26.41(2) | 3.373(3) | 1.80 |
| 30 | 27.26(2) | 3.269(3) | 17.77 |
| 31 | 27.734(4) | 3.2140(5) | 62.18 |
| 32 | 28.30(3) | 3.151(3) | 8.35 |
| 33 | 28.749(18) | 3.1028(19) | 2.53 |
| 34 | 30.08(2) | 2.968(2) | 7.84 |
| 35 | 30.43(2) | 2.935(2) | 5.06 |
| 36 | 30.71(3) | 2.909(2) | 3.26 |
| 37 | 31.09(3) | 2.874(3) | 4.69 |
| 38 | 31.47(2) | 2.840(2) | 4.46 |
| 39 | 31.915(7) | 2.8018(6) | 3.82 |
| 40 | 32.421(14) | 2.7593(11) | 2.56 |
| 41 | 33.813(15) | 2.6488(11) | 2.90 |
| 42 | 34.23(3) | 2.6177(19) | 3.18 |
| 43 | 34.56(2) | 2.5934(15) | 2.75 |
| 44 | 35.65(3) | 2.516(2) | 2.32 |

### Example 26 - Summary on cocrystallization experiments

(R)-Baclofen obtained as in examples 1 to 3 was used for cocrystal screening.

A preliminary study of (R)-Baclofen solubility was performed using different solvents (18 solvents) in order to design a proper experimental space to obtain or detect a new crystalline form.

Solubility test were performed with 25 mg of (R)-Baclofen in a test tube with screw cap and magnetic stirring. The solvent was added in small portions of 2 volumes (mg/ml) at room temperature up to 40 volumes. After each addition, the sample was stirred for 2-5 minutes. If after addition of 40 volumes of solvent the solid did not reach complete dissolution at room temperature, the suspension was heated to reflux.

The solubility results are summarized in Table 13:

**Table 13: Solubility results for (R)-Baclofen in different solvents**

| Sample No. | Solvent | Solubility | Conditions | | XRPD |
|---|---|---|---|---|---|
| | | | Volume | Temperature | |
| 1 | DMSO | Soluble | | | (R)-Baclofen crystal form B |
| 2 | DMF | | | | (R)-Baclofen DMF solvate |

| Sample No. | Solvent | Solubility | Conditions | | XRPD |
|---|---|---|---|---|---|
| | | | Volume | Temperature | |
| 3 | Water | | | | (R)-Baclofen crystal form A |
| 4 | Ethyl acetate | | | | (R)-Baclofen crystal form A |
| 5 | THF | | | | |
| 6 | Acetonitrile | | 40 | Reflux | |
| 7 | Methanol | | | | |
| 8 | Ethanol | Insoluble | | | |
| 9 | Isopropyl alcohol | | | | (R)-Baclofen crystal form B |
| 10 | Isobutyl acetate | | | | |
| 11 | Acetone | | | | |
| 12 | Methyl isobutyl ketone | | | | |
| 13 | Dioxane | | | | |
| 14 | Tert-butyl methyl ether | | | | |
| 15 | Dichloromethane | | | | |
| 16 | Heptane | | | | |
| 17 | Toluene | | | | |
| 18 | cyclohexane | | | | |

The above results allow to conclude that (R)-Baclofen has low solubility in water and majority of commonly used organic solvents:
- (R)-Baclofen is soluble in 40 volume at reflux temperature in DMSO, DMF and water (samples 1-3);
- (R)-Baclofen is insoluble in 40 volume in the other tested solvents even at reflux temperature (samples 4-18).

Cocrystal screening involved 2 techniques (slurrying and grinding), 18 solvents and (R)-Baclofen /coformer molar ratios of 1:1, 1:2, 1:3, 1:5 and 2:3.

The selected coformer's used for cocrystal screening are summarized in Table 14:

**Table 14: List of screened coformers**

| **Coformer** | **CAS Number** |
|---|---|
| Benzoic acid | 65-85-0 |
| Cinnamic acid | 140-10-3 |
| Gentisic acid | 490-79-9 |
| 4-aminobenzoic acid | 150-13-0 |
| Vanillic acid | 121-34-6 |
| p-Anisic acid | 100-09-4 |
| Citric acid | 77-92-9 |
| Gallic acid | 149-91-7 |
| L-Ascorbic acid | 50-81-7 |
| Fumaric acid | 110-17-8 |
| Salicylic acid | 69-72-7 |
| Vanillin | 121-33-5 |
| Maltol | 118-71-8 |
| Ferulic acid | 1135-24-6 |

Combining the above parameters, 237 representative experiments were performed. As a result, new cocrystal forms were detected by XRPD and the results are summarized in Table 15:

**Table 15: New cocrystals of (R)-Baclofen**

| **Coformer** | **Form** |
|---|---|
| Benzoic acid | Form II |
| Cinnamic acid | Forms III and IV |
| Fumaric acid | Form VII |
| Salicylic acid | Form VIII |
| Vanillin | Form IX |
| Ferulic acid | Form XII |

### Example 27 -Crystalline stability study of (R)-Baclofen Cocrystals

Crystalline stability study of the new cocrystals found during the screening was performed under accelerated ICH storage conditions (40°C, 75±5 % RH) for 1 week and 4 weeks. The results are summarized in the Table 16:

**Table 16: Crystalline stability of cocrystals**

| Form | XRPD (crystallinity) | Coformer [pKa] | ΔpKa | 1H NMR molar ratio (R)-Baclofen /coformer | Crystalline stability study | Crystalline stability study |
|---|---|---|---|---|---|---|
| | | | | | 40 °C/ 75 ± 5 % RH | 40 °C/ 75 ± 5 % RH |
| | | | | | After 1 week | After 4 weeks |
| Form II | Moderate | Benzoic acid [pKa 4.10] | -0.3 | 2:1 | stable | Stable |
| Form III | Good | Cinnamic acid [pKa 4.10] | -0.60 | 1:1 | Stable | Stable |
| Form IV | Good | | | | Stable | Stable |
| Form VII | Good | Fumaric acid [pKa 3.03] | 0.77 | 2:1 | Unstable | Unstable |
| Form VIII | Good | Salicylic acid [pKa 2.97] | 0.83 | 1:1 | Stable | Stable |
| Form IX | Good | Vanillin [pKa 7.5] | -3.70 | 2:1 | Unstable | Unstable |
| Form XII | Good | Ferulic acid [pKa 4.26] | | 1:1 | Stable | Stable |

XRPD results show that the (R)-Baclofen-cinnamic acid cocrystal Form III, (R)-Baclofen-cinnamic acid cocrystal Form IV, (R)-Baclofen-benzoic acid cocrystal Form II, (R)-Baclofen-salicylic acid cocrystal Form VIII and (R)-Baclofen-ferulic acid cocrystal Form XII remain stable after 4 weeks at 40°C/ 75 ± 5 % RH.

### Example 28 - Chemical stability study of (R)-Baclofen cocrystals

Chemical stability study of the new cocrystals (cocrystals Form III, Form IV, Form II, Form VIII and Form XII) was performed under accelerated ICH storage conditions (40°C,75±5 % RH). Samples were taken periodically (0 (start), after 1, 2, and 4 weeks) and analyzed by HPLC.

Retention time of (R)-Baclofen under conditions described in the Section "Measuring instruments, conditions and protocols" above, is about 13 min and the relative retention times (RRT) of known impurities are given below.

| | |
|---|---|
| - (3RS)-5-amino-3-(4-chlorophenyl)-5-oxopentanoic acid (Impurity B) | : RRT 0.3 |
| - 3-(4-chlorophenyl)glutaric acid (Impurity C) | : RRT 0.4 |
| - (4RS)-4-(4-chlorophenyl)pyrrolidine-2-one (impurity A) | : RRT 0.7 |

Impurities detected in cinnamic acid

**Table 17: Cinnamic acid impurities**

| Coformer | Identification | RT | RRT | % a/a |
|---|---|---|---|---|
| Cinnamic acid | Cinnamic acid (CA) | 7.5 | 0.56 | 99.91 |
| | Impurity CA1 | 6.3 | 0.47 | 0.03 |
| | Impurity CA2 | 9.2 | 0.69 | 0.04 |
| | Impurity CA3 | 13.5 | 1.01 | 0.02 |

Impurities detected in benzoic acid

**Table 18: Benzoic acid impurities**

| Coformer | Identification | RT | RRT | % a/a |
|---|---|---|---|---|
| Benzoic acid | Benzoic acid (BA) | 4.53 | 0.40 | 100.0% |

Impurities detected in salicylic acid

**Table 19: Salicylic acid impurities**

| Coformer | Identification | RT | RRT | % a/a |
|---|---|---|---|---|
| salicylic acid | Salicylic acid (SA) | 5.23 | 0.47 | 99.95% |
| | Impurity SA1 | 3.70 | 0.33 | 0.05% |

Impurities detected in ferulic acid

**Table 20: Ferulic acid impurities**

| Coformer | Identification | RT | RRT | % a/a |
|---|---|---|---|---|
| Ferulic acid | Ferulic acid (FA) | 4.4 | 0.33 | 99.83 |
| | Impurity FA1 | 3.9 | 0.29 | 0.07 |
| | Impurity FA2 | 7.0 | 0.52 | 0.03 |
| | Impurity FA3 | 9.2 | 0.69 | 0.07 |

(R)-Baclofen-cinnamic acid cocrystal Form III :

**Table 21: HPLC Purity of cocrystal Form III**

| Impurities (HPLC % a/a) | | | |
|---|---|---|---|
| Impurity B | Impurity C | Impurity CA1 | Impurity A+Impurity CA2 |
| RRT 0.3 | RRT 0.4 | RRT 0.48 | RRT 0.7 |
| ND | ND | 0.03 | 0.04 |

| | | | |
|---|---|---|---|
| ND : Not detected | | | |

Cocrystal Form III stability study at 40 °C / 75 ± 5 % RH - summary of HPLC analysis

**Table 22: Chemical stability of cocrystal Form III**

| Time (Week) | Impurities (HPLC % a/a) | | | |
|---|---|---|---|---|
| | Impurity B | Impurity C | Impurity CA1 | Impurity A+Impurity CA2 |
| | RRT 0.3 | RRT 0.4 | RRT 0.48 | RRT 0.7 |
| 0 | ND | ND | 0.03 | 0.04 |
| 1 | ND | ND | 0.03 | 0.04 |
| 2 | ND | ND | 0.03 | 0.04 |
| 4 | ND | ND | 0.03 | 0.04 |

(R)-Baclofen-cinnamic acid cocrystal Form IV :

**Table 23: HPLC purity of cocrystal Form IV**

| Impurities (HPLC % a/a) | | | |
|---|---|---|---|
| Impurity B | Impurity C | Impurity CA1 | Impurity A+Impurity CA2 |
| RRT 0.3 | RRT 0.4 | RRT 0.48 | RRT 0.7 |
| ND | ND | ND | 0.03 |

Cocrystal Form IV stability study at 40 °C / 75 ± 5 % RH - summary of HPLC analysis

**Table 24: Chemical stability of cocrystal Form IV**

| Time (Week) | Impurities (HPLC % a/a) | | | |
|---|---|---|---|---|
| | Impurity B | Impurity C | Impurity CA1 | Impurity A+ Impurity CA2 |
| | RRT 0.3 | RRT 0.4 | RRT 0.48 | RRT 0.7 |
| 0 | ND | ND | ND | 0.03 |
| 1 | ND | ND | ND | 0.03 |
| 2 | ND | ND | ND | 0.03 |
| 4 | ND | ND | ND | 0.03 |

(R)-Baclofen-benzoic acid cocrystal Form II :

**Table 25: HPLC Purity of cocrystal Form II**

| Impurities (HPLC % a/a) | | |
|---|---|---|
| Impurity B | Impurity C | Impurity A |
| RRT 0.3 | RRT 0.4 | RRT 0.7 |
| ND | ND | 0.02 |

Cocrystal Form II stability study at 40 °C / 75 ± 5 % RH - summary of HPLC analysis

**Table 26: Chemical stability of cocrystal Form II**

| Time (Week) | Impurities (HPLC % a/a) | | |
|---|---|---|---|
| | Impurity B | Impurity C | Impurity A |
| | RRT 0.3 | RRT 0.4 | RRT 0.7 |
| 0 | ND | ND | 0.02 |
| 1 | ND | ND | 0.02 |
| 2 | ND | ND | 0.02 |
| 4 | ND | ND | 0.02 |

(R)-Baclofen-salicylic acid cocrystal Form VIII :

**Table 27: HPLC purity of Form cocrystal VIII**

| Impurities (HPLC % a/a) | | | |
|---|---|---|---|
| Impurity B | Impurity SA1 | Impurity C | Impurity A |
| RRT 0.3 | RRT 0.33 | RRT 0.4 | RRT 0.7 |
| ND | 0.05 | ND | 0.04 |

Cocrystal Form VIII stability study at 40 °C / 75 ± 5 % RH - summary of HPLC analysis

**Table 28: Chemical stability of cocrystal Form VIII**

| Time (Week) | Impurities (HPLC % a/a) | | | |
|---|---|---|---|---|
| | Impurity B | Impurity SA1 | Impurity C | Impurity A |
| | RRT 0.3 | RRT 0.33 | RRT 0.4 | RRT 0.7 |
| 0 | ND | 0.05 | ND | 0.04 |
| 1 | ND | 0.05 | ND | 0.04 |
| 2 | ND | 0.05 | ND | 0.04 |
| 4 | ND | 0.05 | ND | 0.04 |

(R)-Baciofen-ferulic acid cocrystal Form XII :

**Table 29: HPLC purity of cocrystal Form XII**

| Impurities (HPLC % a/a) | | | |
|---|---|---|---|
| Impurity B + Impurity FA1 | Impurity C | Impurity FA2 | Impurity A+ Impurity FA3 |
| RRT 0.3 | RRT 0.42 | RRT 0.53 | RRT 0.7 |
| 0.07 | ND | ND | 0.05 |

Cocrystal Form XII stability study at 40 °C / 75 ± 5 % RH - summary of HPLC analysis

**Table 30: Chemical stability of cocrystal Form XII**

| Time (Week) | Impurities (HPLC % a/a) | | | |
|---|---|---|---|---|
| | Impurity B + Impurity FA1 | Impurity C | Impurity CA1 | Impurity A+ Impurity CA2 |
| | RRT 0.3 | RRT 0.42 | RRT 0.53 | RRT 0.7 |
| 0 | 0.07 | ND | ND | 0.05 |
| 1 | 0.07 | ND | ND | 0.04 |
| 2 | 0.08 | ND | ND | 0.05 |
| 4 | 0.07 | ND | ND | 0.05 |

HPLC results show that the (R)-Baclofen-cinnamic acid cocrystal Form III, (R)-Baclofen-cinnamic acid cocrystal Form IV, (R)-Baclofen-benzoic acid cocrystal Form II, (R)-Baclofen-salicylic acid cocrystal Form VIII and (R)-Baclofen-ferulic acid cocrystal Form XII remain stable during 0,1, 2, and 4 weeks at 40°C/ 75 ± 5 % RH.

### Example 29 - Aqueous solubility of (R)-Baclofen cocrystals

**Table 31: Aqueous solubility of (R)-Baclofen crystals**

| | (R)-Baclofen Crystal Form | |
|---|---|---|
| Solvent | Crystal Form A | Crystal Form B |
| | Sparingly soluble | Sparingly soluble |
| Water | 0.8 g/100 ml | 1 g/100 ml |

**Table 32: Aqueous solubility of (R)-Baclofen cocrystals**

| Solvent | (R)-Baclofen cocrystals | | | | |
|---|---|---|---|---|---|
| | Cocrystal Form III | Cocrystal Form IV | Cocrystal Form II | Cocrystal Form VIII | Cocrystal Form XII |
| | Freely Soluble | Freely Soluble | Soluble | Freely Soluble | Soluble |
| Water | 10 g /100 ml | 10 g /100 ml | 3.33 g /100 ml | 10 g /100 ml | 3.33 g/100 ml |

### Example 30 - ¹H-NMR analysis

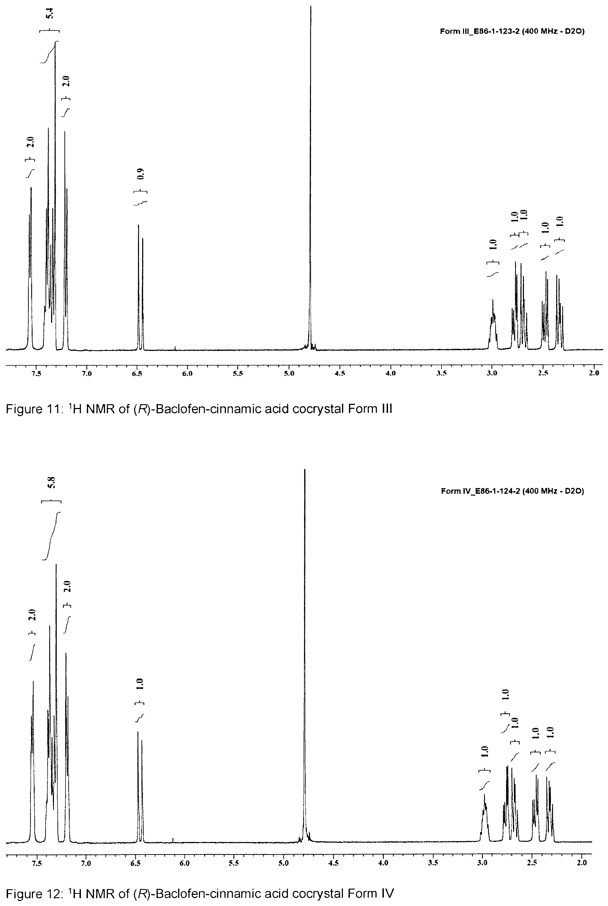

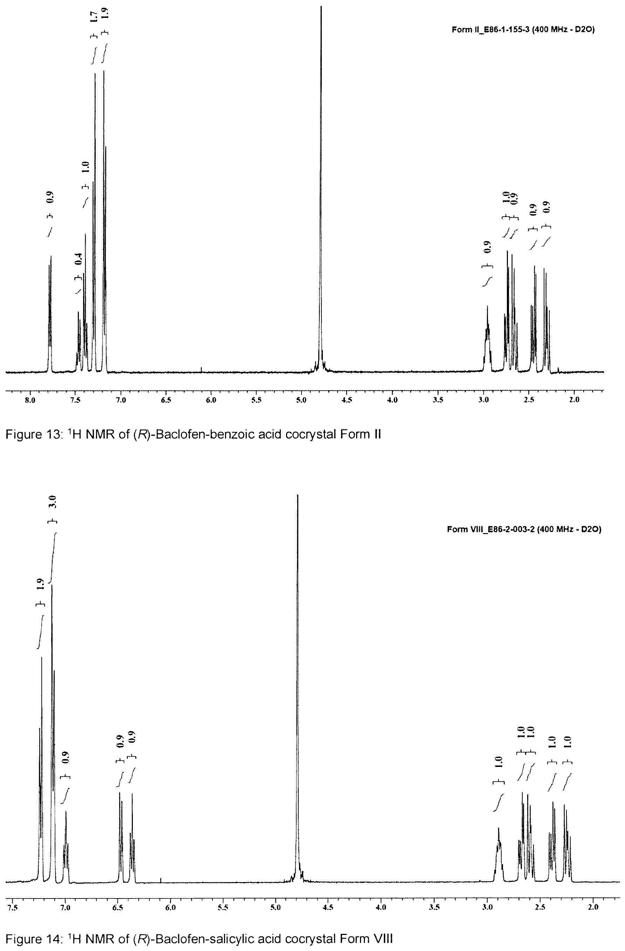

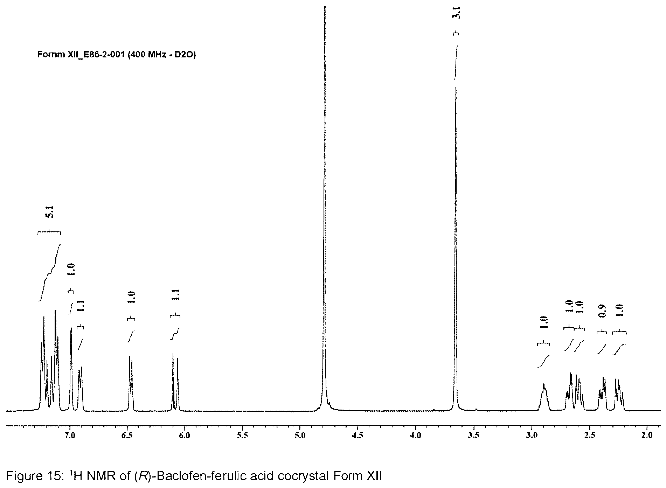

¹H-NMR analyses of (i) (R)-Baclofen-cinnamic acid cocrystal Form III (Figure 11), (ii) (R)-Baclofen-cinnamic acid cocrystal Form IV (Figure 12), (iii) (R)-Baclofen-benzoic acid cocrystal Form II (Figure 13), (iv) (R)-Baclofen-salicylic acid cocrystal Form VIII (Figure 14), and (v) (R)-Baclofen-ferulic acid cocrystal Form XII (Figure 15) were performed using the conditions set forth below.

Proton nuclear magnetic resonance spectra were recorded in deuterium oxide (D₂O).

Spectra were acquired for samples of 5-10 mg dissolved in 0.7ml of D₂O. Few drops of deuterated sodium hydroxide solution (NaOD, 40 % w/w in D₂O) were added to D₂O to achieve a complete dissolution. Spectra were recorded using a Varian Mercury 400 MHz spectrometer equipped with a broadband probe ATB 1H/19F/X of 5 mm.

## Claims

1. A cocrystal of (R)-Baclofen with a coformer.

2. The cocrystal according to claim 1, wherein the coformer is selected from the group consisting of cinnamic acid, benzoic acid, salicylic acid and ferulic acid.

3. The cocrystal according to claim 1 or 2, wherein the coformer is cinnamic acid.

4. The cocrystal according to claim 3, wherein the molar ratio of (R)-Baclofen to cinnamic acid is about 1:1.

5. The cocrystal of claim 3 or 4, wherein it is (*R*)-Baclofen-cinnamic acid cocrystal Form III having an X-ray powder diffraction pattern with peaks at 2-theta angles of 4.60±0.2 degrees, 9.03±0.2 degrees, 13.51±0.2 degrees, 17.99±0.2 degrees, 19.11±0.2 degrees, 19.77±0.2 degrees, 20.65±0.2 degrees, and 23.90±0.2 degrees when using Cu-Ka radiation.

6. The cocrystal according to claim 5, wherein the cocrystal has a single endothermic peak in a differential scanning calorimetry plot at 163.5± 1 °C.

7. A process for preparing (*R*)-Baclofen-cinnamic acid cocrystal Form III according to claim 5 or 6, wherein said process comprises the following steps:
a) contacting (R)-Baclofen and cinnamic acid in a molar ratio of (R)-Baclofen to cinnamic acid within a range 1:1-1:5 with a solvent selected from water, acetonitrile, methyl isobutyl ketone, heptane, cyclohexane, dimethylformamide, ethyl acetate, methanol, ethanol, isopropanol, dioxane, preferably with ethyl acetate,
b) optionally, seeding a mixture of step a) with pre-existing crystals of Form III,
c) crystallising the (R)-Baclofen-cinnamic acid cocrystal Form III.

8. The cocrystal according to claim 3 or 4, wherein it is (*R*)-Baclofen-cinnamic acid cocrystal Form IV having an X-ray powder diffraction pattern with peaks at 2-theta angles of 5.15±0.2 degrees, 10.27±0.2 degrees, 12.84±0.2 degrees, 13.10±0.2 degrees, 15.42±0.2 degrees, 18.58±0.2 degrees, 21.14±0.2 degrees, 24.53±0.2 degrees, 25.34±0.2 degrees, and 27.14±0.2 degrees, when using Cu-Ka radiation.

9. The cocrystal according to claim 8, wherein the cocrystal has a single endothermic peak in a differential scanning calorimetry plot at 161.2 ± 1°C.

10. A process for preparing (*R*)-Baclofen-cinnamic acid cocrystal Form IV according to claim 8 or 9, wherein said process comprises the following steps:
a) contacting (R)-Baclofen and cinnamic acid in a molar ratio of (R)-Baclofen to cinnamic acid within a range 1:1-1:5 with acetone,
b) optionally, seeding a mixture of step a) with pre-existing crystals of Form IV,
c) crystallising the (*R*)-Baclofen-cinnamic acid cocrystal Form IV.

11. The cocrystal according to claim 1 or 2, wherein the coformer is benzoic acid.

12. The cocrystal according to claim 11, wherein the molar ratio of (R)-Baclofen to benzoic acid is about 2:1.

13. The cocrystal of claim 12 wherein it is (R)-Baclofen-benzoic acid cocrystal Form II having an X-ray powder diffraction pattern with peaks at 2-theta angles of 3.4±0.2 degrees, 7.0±0.2 degrees, 10.9±0.2 degrees, 12.7±0.2 degrees, 16.0±0.2 degrees, 17.2±0.2 degrees, 19.2±0.2 degrees, 19.6±0.2 degrees, 20.0±0.2 degrees, 22.1±0.2 degrees, 23.7±0.2 degrees, 25.0±0.2 degrees and 28.5±0.2 degrees, when using Cu-Ka radiation.

14. The cocrystal according to claim 13, wherein the cocrystal in a differential scanning calorimetry plot has a first endothermic peak with an onset at 103.9 ± 1 °C and a second endothermic peak with an onset at 153.4 ± 1 °C.

15. A process for preparing (R)-Baclofen-benzoic acid cocrystal Form II according to claim 13 or 14, wherein said process comprises the following steps:
a) contacting (R)-Baclofen and benzoic acid in a molar ratio of (R)-Baclofen to benzoic acid within a range 1:1-1:5 with a solvent selected from methyl isobutyl ketone, dioxane, cyclohexane, heptane, acetonitrile, ethyl acetate, methanol, ethanol, isopropanol, preferably with methyl isobutyl ketone,
b) optionally, seeding a mixture of step a) with pre-existing crystals of Form II,
c) crystallising the (*R*)-Baclofen-benzoic acid cocrystal Form II.

16. The cocrystal according to claim 1 or 2, wherein the coformer is salicylic acid.

17. The cocrystal according to claim 16, wherein the molar ratio of (R)-Baclofen to salicylic acid is about 1:1.

18. The cocrystal of claim 17, wherein it is (R)-Baclofen-salicylic acid cocrystal Form VIII having an X-ray powder diffraction pattern with peaks at 2-theta angles of 4.62±0.2 degrees, 7.48±0.2 degrees, 8.29±0.2 degrees, 14.56±0.2 degrees, 15.84±0.2 degrees, 17.26±0.2 degrees, 20.25±0.2 degrees, 23.05±0.2 degrees, 24.54±0.2 degrees, and 25.67±0.2 degrees, when using Cu-Ka radiation.

19. The cocrystal according to claim 18, wherein the cocrystal in a differential scanning calorimetry plot has a first endothermic peak with an onset at 115.7 ± 1 °C and two overlapping endothermic peaks with onsets at 140,7 ± 1 °C and 142,7 ± 1 °C.

20. A process for preparing (R)-Baclofen-salicylic acid cocrystal Form VIII according claim 18 or 19, wherein said process comprises the following steps:
a) contacting (R)-Baclofen and salicylic acid in a molar ratio of (R)-Baclofen to salicylic acid within a range 1:1-1:5 with a solvent selected from methyl isobutyl ketone, dichloromethane, dioxane, toluene, dimethylformamide, heptane, tert-butyl methyl ether, tetrahydrofuran, acetonitrile, isopropanol, ethanol, methanol, acetone, ethyl acetate, preferably with tert-butyl methyl ether,
b) optionally, seeding a mixture of step a) with pre-existing crystals of Form VIII,
c) crystallising the (R)-Baclofen-salicylic acid cocrystal Form VIII.

21. The cocrystal according to claim 1 or 2, wherein the coformer is ferulic acid.

22. The cocrystal of claim 21, wherein the molar ratio of (R)-Baclofen to ferulic acid is about 1:1.

23. The cocrystal of claim 22, wherein it is (R)-Baclofen-ferulic acid cocrystal Form XII having an X-ray powder diffraction pattern with peaks at 2-theta angles of 4.62±0.2 degrees, 7.48±0.2 degrees, 8.29±0.2 degrees, 14.56±0.2 degrees, 15.84±0.2 degrees, 17.26±0.2 degrees, 20.25±0.2 degrees, 23.05±0.2 degrees, 24.54±0.2 degrees, and 25.67±0.2 degrees, when using Cu-Ka radiation.

24. The cocrystal according to claim 23, wherein the cocrystal in a differential scanning calorimetry plot has three overlapping endothermic peaks with extrapolated onsets at 116.7± 1°C, 127.2± 1°C and 135.7± 1 °C.

25. A process for preparing (R)-Baclofen-ferulic acid cocrystal Form XII according to claim 23 or 24, wherein said process comprises the following steps:
a) contacting (R)-Baclofen and ferulic acid in a molar ratio of (R)-Baclofen to ferulic acid within a range 1:1 - 1:5 with a solvent selected from water, methanol, ethanol, isopropanol, acetone and a mixture thereof, preferably in acetone/water mixture,
b) optionally, seeding a mixture of step a) with pre-existing crystals of Form XII,
c) crystallising the (*R*)-Baclofen-ferulic acid cocrystal Form XII.

26. Pharmaceutical composition comprising the cocrystal of any of claims 1-6, 8-9, 11-14, 16-19, 21-24 and at least one pharmaceutically acceptable excipient.

27. The pharmaceutical composition of claim 26, wherein said pharmaceutical composition is formulated into a solid unit dosage form for oral administration, preferably into a tablet.

28. The cocrystal of any of claims 1-6, 8-9, 11-14, 16-19, 21-24 or the pharmaceutical composition of claim 26 or 27 for the treatment of a disease or disorder selected from spasticity due to multiple sclerosis, spinal cord injury or cerebral palsy, and alcoholism.
